# EUROPEAN PATENT SPECIFICATION

(11) **EP 1 501 846 B1**
(45) Date of publication and mention of the grant of the patent: **18.03.2009**
(21) Application number: 03731049.7
(22) Date of filing: 25.04.2003
(51) Int. Cl.: C07H 17/08, A61K 31/7048, A61P 31/04

(54) **TRICYCLIC MACROLIDE ANTIBACTERIAL COMPOUNDS**
TRIZYKLISCHE ANTIBAKTERIELLE MAKROLIDDERIVATE
COMPOSES ANTIBACTERIENS DE MACROLIDES TRICYCLIQUES

(30) Priority: 30.04.2002 US 136796; 26.07.2002 US 205708; 24.04.2003 US 422309
(43) Date of publication of application: 02.02.2005
(73) Proprietor: ABBOTT LABORATORIES, Abbott Park IL 60064-3500 (US)
(72) Inventor: GU, Yugui, Libertyville, IL 60048 (US); MA, Zhenkun, Dallas, TX 75225-4627 (US); YONG, Hong, Grayslake, IL 60030 (US)
(74) Representative: Modiano, Micaela Nadia
(86) International application number: PCT/US2003/012971
(87) International publication number: WO 2003/093289

(56) References cited:
- US-A- 5 866 549

## Description

### FIELD OF THE INVENTION

This invention is directed to compounds which are useful as antibacterials, processes for making the compounds and intermediates used in the processes, compositions containing the compounds, and methods for prophylaxis or treatment of bacterial infections using the compounds.

### BACKGROUND OF THE INVENTION

Because the effectiveness of many drugs currently available public use for prophylaxis or treatment of bacterial infections is compromised by the emergence of drug-resistant bacteria, novel antibacterial compounds would be beneficial for their therapeutic value and their contribution to the antibacterial arts.

US 5866549 discloses 6-O-substituted ketolides having antibacterial activity, pharmaceutical compositions comprising the same, methods of treating bacterial infections by the administration of such compounds and processes for the preparation of the compounds.

### SUMMARY OF THE INVENTION

A first embodiment of this invention, therefore, is directed to compounds which inhibit bacterial growth, and salts the compounds having formula (I) 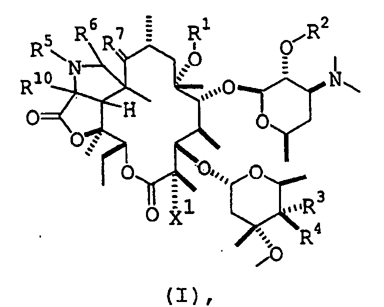 or formula (II), 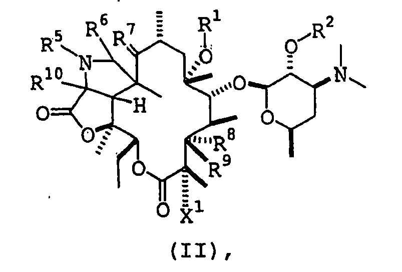 or a pharmaceutically acceptable salt, thereof, in which
R¹ is hydrogen, -R¹¹, -C(O)OR¹¹, -C(O) NH₂, -C(O)NHR¹², or -C(O)NR¹²R¹³:
R² is hydrogen or R^{P}, in which R^{P} is a hydroxyl protecting moiety;
one of R³ or R⁴ is hydrogen and the other is -OH, -OR^{P}, or -OC(O)R¹⁵ ; or
R³ and R⁴ together are =O or -CH₂O-;
R⁵ is hydrogen, -R¹⁹, -C (O) OR¹⁹, -C (O) NH₂, -C (O) NHR²⁰, or -C(O)NR²⁰R²¹;
R⁶ and R¹⁰ are independently hydrogen or -R²³;
R⁷ is =O, =NOH, =NOR^{P}, or =NOR²⁴;
one of R⁸ and R⁹ is hydrogen, and the other is -OH or -OR³²; or
R⁸ and R⁹ together are =O:
R¹¹, R¹⁵, R¹⁹, R²⁴, and R²⁶ are independently alkyl, -(CH₂)alkenyl, -(CH₂)alkynyl, alkyl substituted with one or two or three substituents independently selected from the group consisting of cycloalkyl, halo, aryl, heteroaryl, and heterocyclyl, -(CH₂)alkenyl substituted with one or two or three substituents independently selected from the group consisting of cycloalkyl, halo, aryl, heteroaryl, and heterocyclyl, or -(CH₂)alkynyl substituted with one or two or three substituents independently selected from the group consisting of cycloalkyl, aryl, heteroaryl, and heterocyclyl;
R¹², R¹³, R²⁰, R²¹, R²⁷, and R²⁸ are independently alkyl, cycloalkyl, -(CH₂)alkenyl, -(CH₂)alkynyl, cycloalkyl, aryl, heteroaryl, heterocyclyl, alkyl substituted with one substituent selected from the group consisting of cycloalkyl, aryl, heteroaryl, heterocyclyl, -NH₂, -NHR³⁰, and -NR³⁰R³¹, -(CH₂)alkenyl substituted with one substituent selected from the group consisting of cycloalkyl, aryl, heteroaryl, heterocyclyl, -NH₂, -NHR³⁰, and -NR³⁰R³¹, or -(CH₂)alkynyl substituted with one substituent selected from the group consisting of cycloalkyl, aryl, heteroaryl, heterocyclyl, -NH₂, -NHR³⁰, and -NR³⁰R³¹;
R²³ is alkyl, alkenyl, alkynyl, aryl, heteroaryl, heterocyclyl, alkyl substituted with one or two or three substituents independently selected from the group consisting of cycloalkyl, halo, aryl, heteroaryl, and heterocyclyl, alkenyl substituted with one or two or three substituents independently selected from the group consisting of cycloalkyl, halo, aryl, heteroaryl, and heterocyclyl, or alkynyl substituted with one or two or three substituents independently selected from the group consisting of cycloalkyl, aryl, heteroaryl, and heterocyclyl;
R³⁰ and R³¹ are independently alkyl, cycloalkyl, aryl, heteroaryl, heterocyclyl, -(CH₂)alkenyl, -(CH₂)alkynyl, cycloalkyl, alkyl substituted with one substituent selected from the group consisting of cycloalkyl, aryl, heteroaryl, heterocyclyl, -NH₂, -NH(alkyl), and -N(alkyl)₂, -(CH₂)alkenyl substituted with one substituent selected from the group consisting of cycloalkyl, aryl, heteroaryl, heterocyclyl, -NH₂, -NH(alkyl), and -N(alkyl)₂, or -(CH₂)alkynyl substituted with one substituent selected from the group consisting of cycloalkyl, aryl, heteroaryl, heterocyclyl, -NH₂, =NH(alkyl), and -N(alkyl)₂;
R³² is -R²⁶, -C(O)OR²⁶, -C (O) NH₂, -C (O) NHR²⁷, or -C(O)NR²⁷R²⁸; and
X¹ is hydrogen or fluoride.

A process (not claimed) is disclosed, for making the the compounds having formula (I)-b, 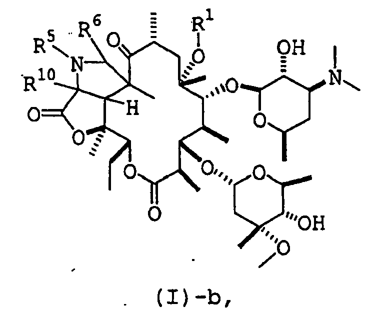 or formula (II)-f, 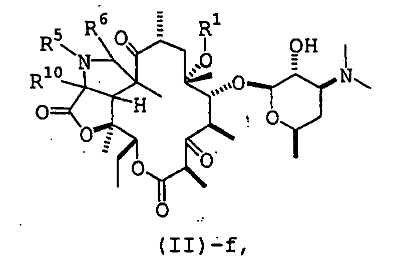 and salts thereof, in which
R¹ is hydrogen, -R¹¹, -C (O) OR¹¹, -C (O) NH₂, -C (O) NHR¹², -C(O)NR¹²R¹³, -CH₂R¹⁴, -C (O) OCH₂R¹⁴, -C(O)NHCH₂R¹⁹, or -C(O)N(CH₂R¹⁴)₂;
R⁵ is hydrogen; -R¹⁹, -C(O) OR¹⁹, -C (O) NH₂, -C(O)NHR²⁰, -C(O)NR²⁰R²¹, -CH₂R²², -C(O)OCH₂R²², -C(O)NHCH₂R²², or -CH₂R²² ;
R⁶ and R¹⁰ are independently hydrogen or -R²³
R¹¹ and R¹⁹ are independently alkyl, - (CH₂) alkenyl, -(CH₂)alkynyl, alkyl substituted with one, two, or three substituents independently selected from the group consisting of cycloalkyl, halo, aryl, heteroaryl, and heterocyclyl, -(CH₂)alkenyl substituted with one or two or three substituents independently selected from the group consisting of cycloalkyl, halo, aryl, heteroaryl, and heterocyclyl, or -(CH₂)alkynyl substituted with one or two or three substituents independently selected from the group consisting of cycloalkyl, aryl, heteroaryl, and heterocyclyl;
R¹², R¹³, R²⁰, and R²¹ are independently alkyl, cycloalkyl, - (CH₂) alkenyl, - (CH₂) alkynyl, aryl, heteroaryl, heterocyclyl, alkyl substituted with one substituent selected from the group consisting of cycloalkyl, aryl, heteroaryl, heterocyclyl, -NH₂, -NHR³⁰, and -NR³⁰R³¹, -(CH₂)alkenyl substituted with one substituent selected from the group consisting of cycloalkyl, aryl, heteroaryl, heterocyclyl, -NH₂, -NHR³⁰, and -NR³⁰R³¹, or - (CH₂) alkynyl substituted with one substituent selected from the group consisting of cycloalkyl, aryl, heteroaryl, heterocyclyl, -NH₂, -NHR³⁰, and -NR³⁰R³¹; or
R¹² and R¹³ together, or R²⁰ and R²¹ together are C₃-C₆-alkylene, C₅-C₆-alkylene interrupted with one moiety selected from the group consisting of -O-, -NH-, -N (alkyl) -, -S-, -S (O) -, and -SO₂-, C₃-C₆-alkylene substituted with one substituent selected from the group consisting of -OH, -O(alkyl), =O, -NH₂, -NHR³⁰, and -NR³⁰R³¹, or C₅-C₆-alkylene interrupted with one moiety selected from the group consisting of -O-, -NH-, -N(alkyl)-, -S-, -S(O)-, and -SO₂- and substituted with one substituent selected from the group consisting of -OH, -O(alkyl), =O, -NH₂, -NHR³⁰, and -NR³⁰R³¹;
R¹⁴ and R²² are independently alkyl interrupted with one or two or three moieties independently selected from the group consisting of -O-, -NH-, -N(alkyl)-, -S-, -S(O)-, and -SO₂- or alkyl interrupted with one or two or three moieties independently selected from the group consisting of -O-, -NH-, -N(alkyl)-, -S-, *-*S(O)*-,* and -SO₂- and substituted with one or two or three substituents independently selected from the group consisting of cycloalkyl, halo, aryl, heteroaryl, heterocyclyl, -OH, =O, -O(alkyl), -NH_{2,} -NHR³⁰, and -NR³⁰R³¹;
R²³ is alkyl, alkenyl, alkynyl, aryl, heteroaryl, heterocyclyl, alkyl substituted with one, two, or three substituents independently selected from the group consisting of cycloalkyl, halo, aryl, heteroaryl, and heterocyclyl, alkenyl substituted with one or two or three substituents independently selected from the group consisting of cycloalkyl, halo, aryl, heteroaryl, and heterocyclyl, alkynyl substituted with one, two, or three substituents independently selected from the group consisting of cycloalkyl, aryl, heteroaryl, and heterocyclyl, alkyl interrupted with one or two or three moieties independently selected from the group consisting of -0-, -NH-, -N(alkyl)-, -S-, -S (O) -, and -SO₂-, alkyl interrupted with one or two or three moieties independently selected from the group consisting of -O-, -NH-, -N(alkyl)-, -S-, -S(O)-, and -SO₂- and substituted with one or two or three substituents independently selected from the group consisting of cycloalkyl, halo, aryl, heteroaryl, heterocyclyl, -OH, =O, -O(alkyl), -NH₂, -NHR³⁰, and -NR³⁰R³¹, alkenyl interrupted with one or two moieties independently selected from the group consisting of -O-, -NH-, -N(alkyl)-, -S-, -S(O)-, and -SO₂-, alkenyl interrupted with one or two moieties independently selected from the group consisting of -O-, -NH-, -N(alkyl)-, -S-, -S(O)-, and -SO₂- and substituted with one or two or three substituents independently selected from the group consisting of cycloalkyl, halo, aryl, heteroaryl, heterocyclyl, -OH, =O, -O(alkyl), -NH₂, -NHR³⁰, and -NR³⁰R³¹, alkynyl interrupted with one or two moieties independently selected from the group consisting of -0-, -NH-, -N(alkyl)-, -S-, -S(O)-, and -SO₂-, or alkynyl interrupted with one or two moieties independently selected from the group consisting of -O-, -NH-, -N(alkyl)-, -S-, -S (O) -, and -SO₂- and substituted with one or two or three substituents independently selected from the group consisting of cycloalkyl, halo, aryl, heteroaryl, heterocyclyl, -OH, =O, -O(alkyl), -NH₂, -NHR³⁰, and -NR³⁰R³¹; and
R³⁰ and R³¹ are independently alkyl, cycloalkyl, aryl, heteroaryl, heterocyclyl, -(CH₂)alkenyl, -(CH₂)alkynyl, cycloalkyl, alkyl substituted with one substituent selected from the group consisting of cycloalkyl, aryl, heteroaryl, heterocyclyl, -NH₂, -NH(alkyl), and -N(alkyl)₂, -(CH₂)alkenyl substituted with one substituent selected from the group consisting of cycloalkyl, aryl, heteroaryl, heterocyclyl, -NH₂, -NH(alkyl), and -N(alkyl)₂, or -(CH₂)alkynyl substituted with one substituent selected from the group consisting of cycloalkyl, aryl, heteroaryl, heterocyclyl, -NH₂, -NH(alkyl), and -N(alkyl)₂;
   or
R³⁰ and R³¹ together are C₃-C₆-alkylene, C₅-C₆-alkylene interrupted with one moiety selected from the group consisting of -O-, -NH-, -N(alkyl)-, -S-, -S(O)-, and -SO₂-, C₃-C₆-alkylene substituted with one substituent selected from the group consisting of -OH, -O(alkyl), =O, -NH₂, -NH(alkyl), and -N(alkyl)₂, or C₅-C₆-alkylene interrupted with one moiety selected from the group consisting of -O-, -NH-, -N(alkyl)-, -S-, -S(O)-, and -SO₂- and substituted with one substituent selected from the group consisting of -OH, -O(alkyl), =O, -NH₂, -NH(alkyl), and -N(alkyl)₂;
the process comprising the steps of:
   (a) reacting a compound having formula (X) 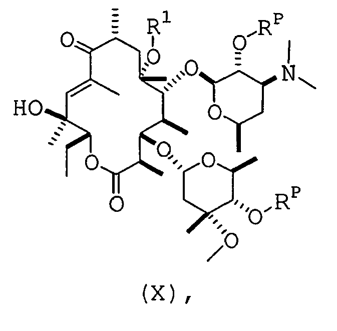 or a compound having formula (IX) 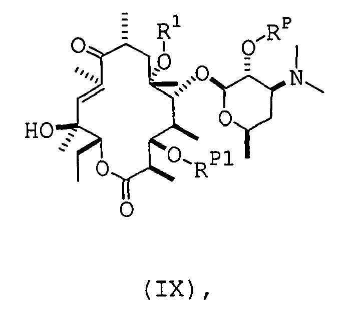 in which R^{P} is a hydroxyl protecting moiety and R^{P1} is trimethylsilyl or triethylsilyl,
      a compound having formula (X²CHR¹⁰CO)₂O,
      in which X² is -Cl or -Br,
      and a second base, with or without
      4-(N,N-dimethylamino)pyridine, to provide a compound having formula (XI) 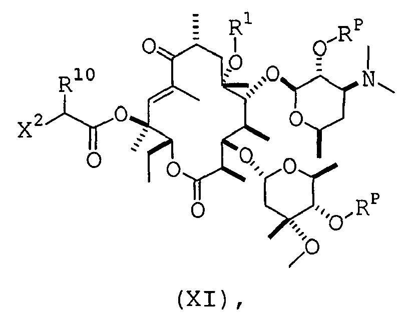 or a compound having formula (XIII) 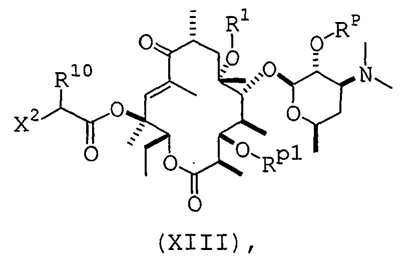 respectively;
   (b) reacting the product of step (a) and a compound having formula R⁵NH₂ to provide a compound having formula (XII) 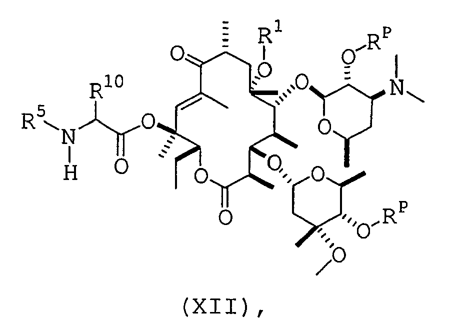 or a compound having formula (XIV) 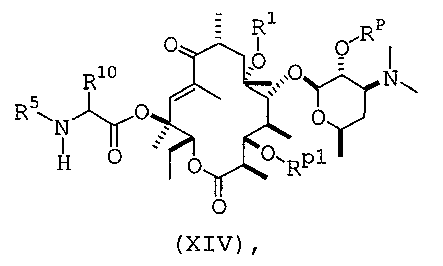 respectively;
   (c) reacting the product of step (b), a compound having formula R⁶CHO, and a first acid, between about 75°C and about 120°C, to provide a compound having formula (I)-a 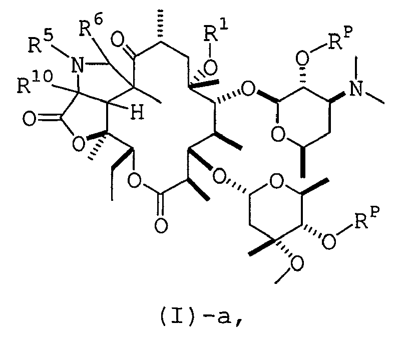 or a compound having formula (XV) 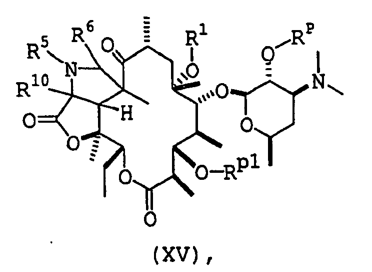 respectively;
   (d) reacting the compound having formula (XV) and a fluorinating agent and reacting the product obtained therefrom and an oxidant, with or without a second base, to provide a compound having formula (II)-c 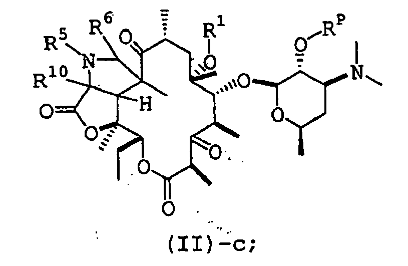 and
      (e)-(1) reacting the compound having formula (I)-a and a deprotecting agent, or
      (e)-(2) reacting the compound having formula (II)-c and a deprotecting agent.

A process (not claimed) is disclosed for making compounds having formula (II)-g 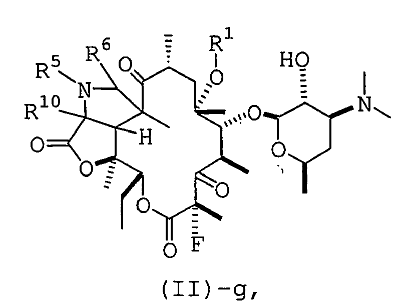 or salts, thereof, in which
R¹ is hydrogen, -R¹¹, -C (O) OR¹¹, -C (O) NH₂, -C (O) NHR¹² , -C (O) NR¹²R¹³, -CH₂R¹⁴, -C(O)OCH₂R¹⁴, -C (O) NHCH₂R¹⁴, or -C (O) N (CH₂R¹⁴) ₂;
R⁵ is hydrogen, -R¹⁹, -C (O) OR¹⁹, -C (O) NH₂, -C (O) NHR²⁰, -C(O)NR²⁰R²¹, -CH₂R²², -C (O) OCH₂R²², -C (O) NHCH₂R²², or -OC(O)N(CH₂R²²)₂;
R⁶ and R¹⁰ are independently hydrogen or -R²³;
R¹¹ and R¹⁹ are independently alkyl, -(CH₂)alkenyl, -(CH₂)alkynyl, alkyl substituted with one, two, or three substituents independently selected from the group consisting of cycloalkyl, halo, aryl, heteroaryl, and heterocyclyl, -(CH₂)alkenyl substituted with one or two or three substituents independently selected from the group consisting of cycloalkyl, halo, aryl, heteroaryl, and heterocyclyl, or -(CH₂)alkynyl substituted with one or two or three substituents independently selected from the group consisting of cycloalkyl, aryl, heteroaryl, and heterocyclyl;
R¹², R¹³, R²⁰, and R²¹ are independently alkyl, cycloalkyl, - (CH₂) alkenyl, - (CH₂) alkynyl, aryl, heteroaryl, heterocyclyl, alkyl substituted with one substituent selected from the group consisting of cycloalkyl, aryl, heteroaryl, heterocyclyl, -NH₂, -NHR³⁰, and -NR³⁰R³¹, -(CH₂)alkenyl substituted with one substituent selected from the group consisting of cycloalkyl, aryl, heteroaryl, heterocyclyl, -NH₂, -NHR³⁰, and -NR³⁰R³¹, or -(CH₂)alkynyl substituted with one substituent selected from the group consisting of cycloalkyl, aryl, heteroaryl, heterocyclyl, -NH₂, -NHR³⁰, and -NR³⁰R³¹; or
R¹² and R¹³ together, or R²⁰ and R²¹ together are C₃-C₆-alkylene, C₅-C₆-alkylene interrupted with one moiety selected from the group consisting of -O-, -NH-, -N(alkyl)-, -S-, -S(O)-, and -SO₂-, C₃-C₆-alkylene substituted with one substituent selected from the group consisting of -OH, -O(alkyl), =O, -NH₂, -NHR³⁰, and -NR³⁰R³¹, or C₅-C₆-alkylene interrupted with one moiety selected from the group consisting of -O-, -NH-, -N(alkyl)-, -S-, -S(O)-, and -SO₂- and substituted with one substituent selected from the group consisting of -OH, -O(alkyl), =O, -NH₂, -NHR³⁰, and -NR³⁰R³¹;
R¹⁴ and R²² are independently alkyl interrupted with one or two or three moieties independently selected from the group consisting of -O-, -NH-, -N(alkyl) -, -S-, -S (O) -, and -SO₂- or alkyl interrupted with one or two or three moieties independently selected from the group consisting of -O-, -NH-, -N(alkyl)-, -S-, -S(O)-, and -SO₂- and substituted with one or two or three substituents independently selected from the group consisting of cycloalkyl, halo, aryl, heteroaryl, heterocyclyl, -OH, =O, -O(alkyl), -NH₂, -NHR³⁰, and -NR³⁰R³¹;
R²³ is alkyl, alkenyl, alkynyl, aryl, heteroaryl, heterocyclyl, alkyl substituted with one, two, or three substituents independently selected from the group consisting of cycloalkyl, halo, aryl, heteroaryl, and heterocyclyl, alkenyl substituted with one or two or three substituents independently selected from the group consisting of cycloalkyl, halo, aryl, heteroaryl, and heterocyclyl, alkynyl substituted with one, two, or three substituents independently selected from the group consisting of cycloalkyl, aryl, heteroaryl, and heterocyclyl, alkyl interrupted with one or two or three moieties independently selected from the group consisting of -O-, -NH-, -N (alkyl) -, -S-, -S (O) -, and -SO₂-, alkyl interrupted with one or two or three moieties independently selected from the group consisting of -O-, -NH-, -N(alkyl)-, -S-, -S(O)-, and -SO₂- and substituted with one or two or three substituents independently selected from the group consisting of cycloalkyl, halo, aryl, heteroaryl, heterocyclyl, -OH, =O, -O(alkyl), -NH₂, -NHR³⁰, and -NR³⁰R³¹, alkenyl interrupted with one or two moieties independently selected from the group consisting of -O-, -NH-, -N(alkyl) -, -S-, -S(O)-, and -SO₂-, alkenyl interrupted with one or two moieties independently selected from the group consisting of -O-, -NH-, -N(alkyl) -, -S-, -S (O) -, and -SO₂- and substituted with one or two or three substituents independently selected from the group consisting of cycloalkyl, halo, aryl, heteroaryl, heterocyclyl, -OH, =O, -O(alkyl), -NH₂, -NHR³⁰, and -NR³⁰R³¹, alkynyl interrupted with one or two moieties independently selected from the group consisting of -O-, -NH-, -N(alkyl)-, -S-, -S(O)-, and -SO₂-, or alkynyl interrupted with one or two moieties independently selected from the group consisting of -O-, -NH-, -N(alkyl)-, -S-, -S(O)-, and -SO₂- and substituted with one or two or three substituents independently selected from the group consisting of cycloalkyl, halo, aryl, heteroaryl, heterocyclyl, -OH, =O, -O(alkyl), -NH₂, -NHR³⁰, and -NR³⁰R³¹; and
R³⁰ and R³¹ are independently alkyl, cycloalkyl, aryl, heteroaryl, heterocyclyl, -(CH₂)alkenyl, -(CH₂)alkynyl, cycloalkyl, alkyl substituted with one substituent selected from the group consisting of cycloalkyl, aryl, heteroaryl, heterocyclyl, -NH₂, -NH(alkyl), and -N(alkyl)₂, -(CH₂)alkenyl substituted with one substituent selected from the group consisting of cycloalkyl, aryl, heteroaryl, heterocyclyl, -NH₂, -NH(alkyl), and -N(alkyl)₂, or -(CH₂)alkynyl substituted with one substituent selected from the group consisting of cycloalkyl, aryl, heteroaryl, heterocyclyl, -NH₂, -NH(alkyl), and -N(alkyl)₂;
or
R³⁰ and R³¹ together are C₃-C₆-alkylene, C₅-C₆-alkylene interrupted with one moiety selected from the group consisting of -O-, -NH-, -N(alkyl)-, -S-, -S(O) -, and -SO₂-, C₃-C₆-alkylene substituted with one substituent selected from the group consisting of -OH, -O(alkyl), =O, -NH₂, -NH(alkyl), and -N(alkyl)₂, or C₅-C₆-alkylene interrupted with one moiety selected from the group consisting of -O-, -NH-, -N(alkyl)-, -S-, -S(O)-, and -SO₂- and substituted with one substituent selected from the group consisting of -OH, -O(alkyl), =O, -NH₂, -NH(alkyl), and -N(alkyl)₂;
the process comprising the steps of:
(a) reacting a compound having formula (II)-c 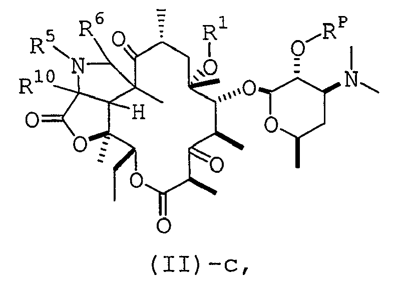 in which R^{P} is a hydroxyl protecting moiety, and a fluorinating agent, with or without a fourth base; and
(b) reacting the product of step (a) and deprotecting agent.

The compounds (not claimed) are disclosed having formula (XI) 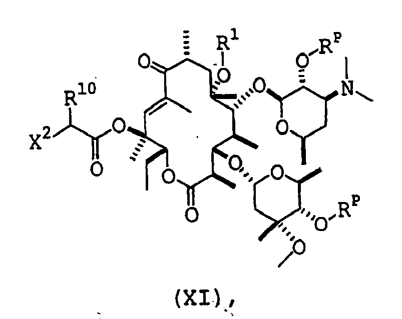 formula (XII), 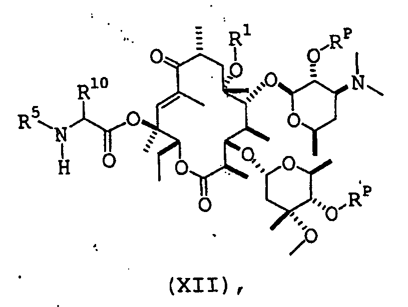 formula (XIII), 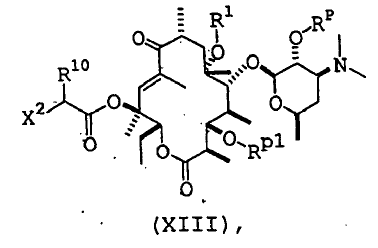 or formula (XIV), 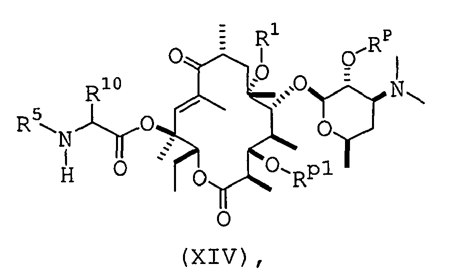 and salts thereof, in which
R¹ is hydrogen, -R¹¹, -C(O)OR¹¹, -C (O) NH₂, -C(O)NHR¹², -C(O)NR¹²R¹³, -CH₂R¹⁴, -C(O)OCH₂R¹⁴, -C(O)NHCH₂R¹⁴, or -C(O)N(CH₂R¹⁴)₂;
R⁵ is hydrogen, -R¹⁹, -C(O)OR¹⁹, -C(O)NH₂, -C(O)NHR²⁰, -C(O)NR²⁰R²¹, -CH₂R²², -C (O) OCH₂R²², -C (O) NHCH₂R²², or -OC(O)N(CH₂R²²)₂;
R¹⁰ is hydrogen or -R²³;
R¹¹ and R¹⁹ are independently alkyl, - (CH₂)alkenyl, -(CH₂)alkynyl, alkyl substituted with one or two or three substituents independently selected from the group consisting of cycloalkyl, halo, aryl, heteroaryl, and heterocyclyl, -(CH₂)alkenyl substituted with one or two or three substituents independently selected from the group consisting of cycloalkyl, halo, aryl, heteroaryl, and heterocyclyl, or -(CH₂)alkynyl substituted with one or two or three substituents independently selected from the group consisting of cycloalkyl, aryl, heteroaryl, and heterocyclyl;
R¹², R¹³, R²⁰, and R²¹ are independently alkyl, cycloalkyl, -(CH₂)alkenyl, -(CH₂)alkynyl, aryl, heteroaryl, heterocyclyl, alkyl substituted with one substituent selected from the group consisting of cycloalkyl, aryl, heteroaryl, heterocyclyl, -NH₂, -NHR³⁰, and -NR³⁰R³¹, -(CH₂)alkenyl substituted with one substituent selected from the group consisting of cycloalkyl, aryl, heteroaryl, heterocyclyl, -NH₂, -NHR³⁰, and -NR³⁰R³¹, or -(CH₂)alkynyl substituted with one substituent selected from the group consisting of cycloalkyl, aryl, heteroaryl, heterocyclyl, -NH₂, -NHR³⁰, and -NR³⁰R³¹; or
R¹² and R¹³ together, or R²⁰ and R²² together are independently C₃-C₆-alkylene, C₅-C₆-alkylene interrupted with one moiety selected from the group consisting of -O-, -NH-, -N(alkyl)-, -S-, -S (O) -, and -SO₂-, C₃-C₆-alkylene substituted with one substituent selected from the group consisting of -OH, -O(alkyl), =O, -NH₂, -NHR³⁰, and -NR³⁰R³¹, or C₅-C₆-alkylene interrupted with one moiety selected from the group consisting of -O-, -NH-, -N(alkyl)-, -S-, -S(O)-, and -SO₂- and substituted with one substituent selected from the group consisting of -OH, -O(alkyl), =O, -NH₂, -NHR³⁰, and -NR³⁰R³¹;
R¹⁴ and R²² are independently alkyl interrupted with one or two or three moieties independently selected from the group consisting of -O-, -NH-, -N(alkyl)-, -S-, -S(O)-, and -SO₂- or alkyl interrupted with one or two or three moieties independently selected from the group consisting of -O-, -NH-, -N(alkyl)-, -S-, -S(O)-, and -SO₂- and substituted with one or two or three substituents independently selected from the group consisting of cycloalkyl, halo, aryl, heteroaryl, heterocyclyl, -OH, =O, -O(alkyl), -NH₂, -NHR³⁰, and -NR³⁰R³¹;
R²³ is alkyl, alkenyl, alkynyl, aryl, heteroaryl, heterocyclyl, alkyl substituted with one or two or three substituents independently selected from the group consisting of cycloalkyl, halo, aryl, heteroaryl, and heterocyclyl, alkenyl substituted with one or two or three substituents independently selected from the group consisting of cycloalkyl, halo, aryl; heteroaryl, and heterocyclyl, alkynyl substituted with one or two or three substituents independently selected from the group consisting of cycloalkyl, aryl, heteroaryl, and heterocyclyl, alkyl interrupted with one or two or three moieties independently selected from the group consisting of -O-, -NH-, -N(alkyl)-, -S-, -S(O)-, and -SO₂-, alkyl interrupted with one or two or three moieties independently selected from the group consisting of -O-, -NH-, -N(alkyl)-, -S-, -S(O)-, and -SO₂- and substituted with one or two or three substituents independently selected from the group consisting of cycloalkyl, halo, aryl, heteroaryl, heterocyclyl, -OH, =O, -O(alkyl), -NH₂, -NHR³⁰, and -NR³⁰R³¹, alkenyl interrupted with one or two moieties independently selected from the group consisting of -O-, -NH-, -N(alkyl)-, -S-, -S(O)-, and -SO₂-, alkenyl interrupted with one or two moieties independently selected from the group consisting of -O-, -NH-, -N(alkyl)-, -S-, -S(O)-, and -SO₂- and substituted with one or two or three substituents independently selected from the group consisting of cycloalkyl, halo, aryl, heteroaryl, heterocyclyl, -OH, =O, -O(alkyl), -NH₂, -NHR³⁰, and -NR³⁰R³¹, alkynyl interrupted with one or two moieties independently selected from the group consisting of -O-, -NH-, -N (alkyl) -, -S-, -S(O)-, and -SO₂-, or alkynyl interrupted with one or two moieties independently selected from the group consisting of -O-, -NH-, -N(alkyl)-, -S-, -S (O) -, and -SO₂- and substituted with one or two or three substituents independently selected from the group consisting of cycloalkyl, halo, aryl, heteroaryl, heterocyclyl, -OH, =O, -O(alkyl), -NH₂, -NHR³⁰, and -NR³⁰R³¹;
R³⁰ and R³¹ are independently alkyl, cycloalkyl, aryl, heteroaryl, heterocyclyl, - (CH₂) alkenyl, - (CH₂) alkynyl, cycloalkyl, alkyl substituted with one substituent selected from the group consisting of cycloalkyl, aryl, heteroaryl, heterocyclyl, -NH₂, -NH(alkyl), and -N(alkyl)₂, -(CH₂)alkenyl substituted with one substituent selected from the group consisting of cycloalkyl, aryl, heteroaryl, heterocyclyl, -NH₂, -NH(alkyl), and -N(alkyl)₂, or -(CH₂)alkynyl substituted with one substituent selected from the group consisting of cycloalkyl, aryl, heteroaryl, heterocyclyl, -NH₂, -NH (alkyl), and -N(alkyl)₂; or
R³⁰ and R³¹ together are C₃-C₆-alkylene, C₅-C₆-alkylene interrupted with one moiety selected from the group consisting of -O-, -NH-, -N(alkyl)-, -S-, -S(O)-, and -SO₂-, C₃-C₆-alkylene substituted with one substituent selected from the group consisting of -OH, -O(alkyl), =O, -NH₂, -NH(alkyl), and -N(alkyl)₂, or C₅-C₆-alkylene interrupted with one moiety selected from the group consisting of -O-, -NH-, -N (alkyl) -, -S-, -S(O)-, and -SO₂- and substituted with one substituent selected from the group consisting of -OH, -O(alkyl), =O, -NH₂, -NH(alkyl), and -N(alkyl)₂:
R^{P} is (methyl)carbonyl or (phenyl)carbonyl:
R^{P1} is trimethylsilyl or triethylsilyl; and
X² is chloride or bromide.

A second embodiment of this'invention is directed to compositions which are useful for prophylaxis or treatment of bacterial infections in a fish or a mammal comprising a therapeutically effective amount of one or more of the compounds of the first embodiment and an excipient.

A third embodiment of this invention is directed to use of the compounds of the first embodiment for preparation of a medicament for prophylaxis or treatment of bacterial infections.

### DETAILED DESCRIPTION OF THE INVENTION

Compounds of this invention, also referred to as "the compounds," comprise both.fixed and variable moieties, which variable moieties are identified by a capital letter and accompanying numerical and/or alphabetical superscript, and for which the following terms have the meanings indicated.

"Alkenyl" means monovalent, straight-chain and branched-chain hydrocarbon moieties, having two to eight carbon atoms and at least one carbon-carbon double bond, attached through a carbon atom.

Examples of alkenyl moieties include but-1,3-dienyl, butenyl, but-2-enyl, ethenyl, 1-ethylhexen-2-yl, hex-3-enyl, 1-methylbutenyl, 2-methylbutenyl, 1-methylbut-2-enyl, 1-methylbut-1,3-dienyl, pentenyl, pent-2-enyl, pent-3-enyl, and propenyl.

"Alkyl" means monovalent, saturated, straight-chain and branched-chain hydrocarbon moieties, having one to six carbon atoms, attached through a carbon atom.

Examples of alkyl moieties include butyl, 1,1,-dimethylethyl, 1,1-dimethylpropyl, 1,2-dimethylpropyl, ethyl, 1-ethylpropyl, 2-ethylpropyl, hexyl, methyl, 2-methylpropyl, 3-methylbutyl, 1-methylpentyl, 2-methylpent-3-yl, and pentyl.

"Alkylene" means divalent, saturated, straight-chain and branched-chain hydrocarbon moieties, having one to eight carbon atoms, attached through carbon atoms.

Examples of alkylene moieties include butylene, 1,1,-dimethylethylene, 1,1-dimethylpropylene, 1,2-dimethylpropylene, ethylene, 1-ethylpropylene, 2-ethylpropylene, hexylene, methylene, 2-methylpropylene, 3-methylbutylene, 1-methylpentylene, 2-methyl-2-ethylpropylene, and pentylene.

"Alkynyl" means monovalent, straight-chain and branched-chain hydrocarbon moieties, having two to six carbon atoms and at least one carbon-carbon triple bond, attached through a carbon atom.

Examples of alkynyl moieties include ethynyl (acetylenyl), pentynyl, pent-2-ynyl, pent-3-ynyl, pent-4-ynyl, 1-methylbut-2-ynyl, 2-methylbut-3-ynyl, hexynyl, hex-2-ynyl, hex-3-ynyl, hex-4-ynyl, 1-methyl-pent-2-ynyl, 1-methylenepent-3-ynyl, 1-methyl-pent-2,4-diynyl, and prop-2-ynyl (propargyl).

"Aryl" means monovalent, unsubstituted and substituted phenyl moieties, attached through a carbon atom and unfused or fused with another phenyl moiety or a cycloalkyl, cycloalkenyl, heteroaryl, heterocyclyl, naphthyl, or saturated part of an indanyl moiety.

Examples of phenyl moieties fused with phenyl, naphthyl, or the saturated part of an indanyl moieties are unsubstituted and substituted naphth-(1- or 2- or 3- or 4-) yl, anthracen-(1- or 2- or 3- or 4-)yl, and fluoren-(1- or 2- or 3- or 4-)yl, respectively.

Examples of phenyl moieties fused with cycloalkyl moieties are unsubstituted and substituted indan-(4- or 5- or 6- or 7-)yl and 1,2,3,4-tetrahydronaphth-(5- or 6- or 7- or 8-)yl.

Examples of phenyl moieties fused with cycloalkenyl moieties are unsubstituted and substituted inden-(4- or 5- or 6- or 7-)yl, 1,2-dihydronaphth-(5- or 6- or 7- or 8-)yl and 1,2-dihydronaphth-(5- or 6- or 7- or 8-)yl.

Examples of phenyl moieties fused with heteroaryl moieties include unsubstituted and substituted benzimidazol-(4- or 5- or 6- or 7-)yl, 1-benzofuran-(4- or 5- or 6- or 7-yl, 1,2-benzisothiazol-(4- or 5- or 6- or 7-yl, benzthiazol-(4- or 5- or 6- or 7-)yl, 1-benzothiophen-(4- or 5- or 6- or 7-)yl, cinnolin-(5- or 6- or 7- or 8-)yl, indol-(4- or 5- or 6- or 7-)yl, isoquinolin-(5- to 8-)yl, phthalazin-(5- to 8-)yl, quinazolin-(5- to 8-)yl, quinolin-(5- or 6- or 7- or 8-)yl, and quinoxalin-(5- or 6- or 7- or 8-)yl.

Examples of phenyl moieties fused with heterocyclyl moieties include unsubstituted and substituted 1,3-benzodiox-(4- or 5- or 6- or 7-)yl, 1,4-benzodiox-(5- or 6- or 7- or 8-)yl, 1,3-dihydro-2-benzofuran-(4- or 5- or 6- or 7-)yl, 2,3-dihydro-1-benzofuran-(4- or 5- or 6- or 7-)yl, 1,3-dihydro-2-benzothiophen-(4- or 5- or 6- or 7-)yl, 2,3-dihydro-1-benzothiophen-(4- or 5- or 6- or 7-)yl, and indolin-(4- or 5- or 6- or 7-)yl.

"Cycloalkyl" means monovalent, unsubstituted and substituted, saturated cyclic hydrocarbon moieties, having three to six carbon atoms, attached through a carbon atom.

Examples of cycloalkyl moieties are unsubstituted and substituted cyclopropyl, cyclobutyl, cyclopentyl, and cyclohexyl.

"Cycloalkenyl" means means monovalent, unsubstituted and substituted, cyclic hydrocarbon moieties having four to six carbon atoms and at least one carbon-carbon double bond, attached through a carbon atom.

Examples of cycloalkenyl moieties are unsubstituted and substituted 1,3-cyclohexadienyl, 1,4-cyclohexadienyl, cyclohexenyl, cyclopentadienyl, and cyclopentenyl.

"Halo" means fluoro (-F), chloro (-C1), bromo (-Br), and iodo (-I) moieties.

"Heteroaryl" means (1) monovalent, aromatic, unsubstituted and substituted five-membered ring moieties having two double bonds and (a) one oxygen or one sulfur atom, (b) one, two, three, or four nitrogen atoms, or (c) one or two nitrogen atoms and one oxygen or one sulfur atom, in which, for'(a), (b) and (c), the remaining atoms are carbon atoms and the rings themselves may be attached through a carbon atom or a nitrogen atom; and (2) monovalent six-membered ring moieties having three double bonds and one or two or three nitrogen atoms and the remaining atoms are carbon atoms, and the rings themselves are attached through a carbon atom; in which the heteroaryl moieties (1) and (2) are unfused or fused with another heteroaryl moiety or an aryl moiety.

Examples of five-membered heteroaryl moieties are unsubstituted and substituted furanyl, imidazolyl, isothiazolyl, isoxazolyl, 1,2,3-oxadiazolyl, oxazolyl, pyrazolyl, pyrrolyl, tetrazolyl, 1,3,4-thiadiazolyl, thiazolyl, thiophenyl (thienyl), 1H-tetrazolyl, 2H-tetraazolyl, and 1,2,3-triazolyl.

Examples of five-membered heteroaryl moieties fused with aryl moieties include unsubstituted and substituted benzimidazol-(1- or 2-)yl, 1-benzofuran-(2- or 3-)yl, 1,2-benzisothiazol-3-yl, benzthiazol-2-yl, 1-benzothiophen-(2- or 3-)yl, cinnolin-(3- or 4-)yl, indol-(1- or 2- or 3-)yl, isoquinolin-(1- or 3- or 4-)yl, phthalazin-(1- or 4-)yl, quinazolin-(2- or 4-)yl, quinolin-(2- or 3- or 4-)yl, and quinoxalin-(2- or 3-)yl.

Examples of five-membered heteroaryl moieties fused with other five-membered heteroaryl moieties include unsubstituted and substituted (1,3)thiazolo(4,5-d)(1,3)oxazolyl, (1,3)thiazolo(4,5-d) (1,3)thiazolyl, thieno(3,2-d)(1,3)oxazolyl, thieno(3,2-d)(1,3)thiazolyl, and thieno(2,3-b)thiophenyl.

Examples of five-membered heteroaryl moieties fused with six-membered heteroaryl moieties include unsubstituted and substituted furo(2,3-b)pyridin-(2- or 3-)yl, 3H-imidazo(4,5-b)pyridin-(2- or 3-)yl, (1,3)thiazolo(4,5-b)pyrazin-2-yl, (1,3)thiazolo(4,5-b)pyridin-2-yl, and thieno(2,3-b)pyridin-(2- or 3-)yl.

Examples of six-membered heteroaryl moieties are unsubstituted and substituted pyrazinyl, pyridazinyl, pyridyl, pyrimidinyl, and 1,3,5-triazinyl.

Six-membered heteroaryl moieties fused with aryl moieties include unsubstituted and substituted cinnolin-(3- or 4-)yl, isoquinolin-(1 or 3- or 4-)yl, phthalazin-(1 or 4-)yl, quinazolin-(2- or 4-)yl, quinolin-(2- or 3- or 4-)yl, and quinoxalin-(2- or 3-)yl.

Six-membered heteroaryl moieties fused with five-membered heteroaryl moieties include unsubstituted and substituted furo(2,3-b)pyridin-(4- or 5- or 6-)yl, 3H-imidazo(4,5-b)pyridin-(5- or 6- or 7-)yl, (1,3)thiazolo(4,5-b)pyrazin-(5- or 6-)yl, (1,3)thiazolo(4,5-b)pyridin-(5- or 6- or 7-)yl, and thieno(2,3-b)pyridin-(4- or 5- or 6-)yl.

Six-membered heteroaryl moieties fused with other six-membered heteroaryl moieties include unsubstituted and substituted 1,5-naphthyridinyl, 1,7-naphthyridinyl, 1,8-naphthyridinyl, pteridinyl, pyridazino(4,5-d)pyridazinyl, pyrido(2,3-d)pyridazinyl, and pyrido(3,4-d)pyridazinyl.

"Heterocyclyl" means (a) monovalent, non-aromatic, unsubstituted and substituted four-membered ring moieties having one nitrogen, oxygen, or sulfur atom and the remaining atoms are carbon atoms, zero double bonds, attached through a carbon atom or a nitrogen atom, (b) monovalent, non-aromatic, unsubstituted and substituted five-membered ring moieties having one or two nitrogen, oxygen, or sulfur atoms and the remaining atoms are carbon atoms, and zero or one double bonds, attached through a carbon atom or a nitrogen atom, and (c) monovalent, non-aromatic, unsubstituted and substituted six-membered ring moieties having one or two or three nitrogen, oxygen, or sulfur atoms and the remaining atoms are carbon atoms, and zero, one, or two double bonds, attached through a carbon atom or a nitrogen atom.

Examples of four-membered heterocyclyl moieties are unsubstituted and substituted oxetane, thietane, and azetidine.

Examples of five-membered heterocyclyl moieties include unsubstituted and substituted 1,4-dioxanyl, 1,3-dioxolanyl, imidazolidinyl, 2-imidazolinyl, 4,5-dihydroisoxazolyl, pyrazolidinyl, 2-pyrazolinyl, pyrrolidinyl, 2-pyrrolinyl, 3-pyrrolinyl, and 2H-pyrrolyl.

Examples of six-membered heterocyclyl moieties include unsubstituted and substituted 1,3-dithianyl, 1,4-dithianyl, morpholinyl, piperidinyl, piperazinyl, pyranyl, 2H-pyranyl, 4H-pyranyl, and thiomorpholinyl.

Substituted aryl and heteroaryl moieties are those moieties substituted with one, two, three, four, or five substituents independently selected from the group consisting of alkyl, alkenyl, alkynyl, cycloalkyl, halo, -CN, -OH, -SH, -NH₂, -NO₂, -CF₃, -CH₂CF₃, -CF₂CF₃, -OCF₃, -OCH₂CF₃, -OCF₂CF₃, -OR³⁰ -SR³⁰, -S(O)(alkyl), -SO₂(alkyl), -C(O)H, -C(O)(alkyl), -C(O)OH, -C(O)O(alkyl), -NH(alkyl), -N(alkyl)₂, -C(O)NH₂, -C(O)NH(alkyl), -C(O)N(alkyl)₂, -OC(O)(alkyl), -OC(O)O(alkyl), -OC(O)NH₂, -OC(O)NH(alkyl), -OC(O)N(alkyl)₂, -NHC(O)H, -NHC(O)(alkyl), -NHC(O)O(alkyl), -NHC(O)NH₂, -NHC(O)NH(alkyl), -NHC(O)N(alkyl)₂, -SO₂NH₂, -SO₂NH(alkyl), -SO₂N (alkyl)₂, and R⁴⁰, in which R³⁰ is alkyl or alkyl substituted with one substituent selected from the group consisting of halo, -O(alkyl), and -S(alkyl), and R⁴⁰ is furyl, imidazolyl, indazolidinyl, isoquinolinyl, isothiazolyl, isoxazolyl, morpholinyl, naphthyl, naphthyridyl, 1,2,3-oxadiazolyl, oxazolyl, phenyl, piperidinyl, piperazinyl, pyrazinyl, pyrazolyl, pyridyl, pyrimidinyl, pyrrolidinyl, pyrrolyl, quinazolyl, quinolinyl, quinoxalyl, tetrazolyl, 1,2,3-thiadiazolyl, 1,3,4-thiadiazolyl, thiazolyl, thienyl, 1,2,3-triazolyl, or thiomorpholinyl, in which each R⁴⁰ moiety is unsubstituted or substituted with one or two or three substituents independently selected from the group consisting of alkyl, alkenyl, alkynyl, cycloalkyl, halo, =O, -CN, -OH, -SH, -NO₂, -CF₃, -CH₂CF₃, -CF₂CF₃, -OCF₃, -OCH₂CF₃, -OCF₂CF₃, -O(alkyl), -S(alkyl), -S(O)(alkyl), -SO₂ (alkyl), -C(O)H, -C(O)(alkyl), -C(O)OH, -C(O)O(alkyl), -NH₂, -NH(alkyl), -N(alkyl)₂, -C(O)NH₂, -C(O)NH(alkyl), -C(O)N(alkyl)₂, -OC(O)(alkyl), -OC(O)O(alkyl), -OC(O)NH₂, -OC(O)NH(alkyl), -OC(O)N(alkyl)₂, -NHC(O)H, -NHC(O)(alkyl), -NHC(O)O(alkyl), -NHC(O)NH₂, -NHC(O)NH(alkyl), -NHC(O)N(alkyl)₂, -SO₂NH₂, -SO₂NH(alkyl), and -SO₂N(alkyl)2_{.}

Substituted cycloalkyl, cycloalkenyl, and heterocyclyl moieties are those moieties substituted with one or two or three substituents independently selected from the group consisting of alkyl, phenyl, halo, -CN, -OH, -NH₂, -CF₃, -OR³⁰, -SR³⁰, -S(O)(alkyl), -SO₂(alkyl), -C(O)H, -C(O)(alkyl), -C(O)OH, -C(O)O(alkyl), -NH(alkyl), -N(alkyl)₂, -C(O)NH₂, -C(O)NH(alkyl), and -C(O)N(alkyl)₂, in which the phenyl is unsubstituted or substituted with one or two or three substituents independently selected from the group consisting of halo, -CN, -OH, -NH₂, and -CF₃.

"Hydroxyl protecting moiety" means selectively introducible and removable moieties which protect -OH moieties against undesirable side reactions. Hydroxyl protecting protecting moieties include 4-nitrobenzyloxycarbonyl, 4-bromobenzyloxycarbonyl, 4-methoxybenzyloxycarbonyl, 3,4-dimethoxybenzyloxycarbonyl, tert-butoxycarbonyl, diphenylmethoxycarbonyl, 2,2,2-trichloroethoxycarbonyl, 2,2,2-tribromoethoxycarbonyl, 2-(trimethylsilyl)-ethoxycarbonyl, 2-(phenylsulfonyl)ethoxycarbonyl, allyloxycarbonyl, acetyl, chloroacetyl, dichloroacetyl, trichloroacetyl, trifluoroacetyl, methoxyacetyl, phenoxyacetyl, pivaloyl, propionyl, 2-methylpropionyl, benzoyl, tert-butyl, 2,2,2-trichloroethyl, 2-trimethylsilylethyl, 1,1-dimethyl-2-propenyl, 3-methyl-3-butenyl, para-methoxybenzyl, 3,4-dimethoxybenzyl, diphenylmethyl, triphenylmethyl, tetrahydrofuryl, benzyloxymethyl, 2-methoxyethoxymethyl, 2,2,2-trichloroethoxymethyl, 2-(trimethylsilyl)-ethoxymethyl, methanesulfonyl, para-toluenesulfonyl, trimethylsilyl, triethylsilyl, triisopropylsilyl, diethylisopropylsilyl, tert-butyldimethylsilyl, tert-butyldiphenylsilyl, diphenylmethylsilyl, and tert-butylmethoxyphenylsilyl.

These variable moieties may combine to provide a fourth embodiment of this invention, which embodiment is directed to compounds having formula (I) or formula (II), and pharmaceutically acceptable salts.
thereof, in which R¹ is methyl, prop-2-ynyl, 3-(5-pyridin-2-ylthien-2-yl)prop-2-ynyl , 3-(quinolin-3-yl)prop-2-enyl, 3-(3-pyridin-2-ylisoxazol-5-yl)prop-2-ynyl, or 3-(5-pyrimidin-2-ylthien-2-yl)prop-2-ynyl; R² is hydrogen; R³ is -OH, ((phenyl)carbonyl)oxy, ((methyl)carbonyl)oxy, (trimethylsilyl)oxy, or (triethylsilyl)oxy and R⁴ is hydrogen, or R³ and R⁴ together are =O or -CH₂O-; R⁵ is hydrogen, methyl, ethyl, propyl, prop-2-ynyl, prop-2-enyl, phenylmethyl, 4-methoxyphenylmethyl, or 2,4-dimethoxyphenylmethyl; R⁶ is hydrogen, methyl, ethyl, ethenyl, or phenyl; R⁷ is =O; R⁸ is hydrogen and R⁹ is -OH, or R⁸ and R⁹ together are =O; R¹⁰ is hydrogen, methyl, ethyl, prop-2-ynyl or prop-2-enyl; and X¹ is hydrogen or fluoride.

Specific examples of R¹ moieties for the practice of this invention are methyl, prop-2-ynyl, and 3-(5-pyridin-2-ylthien-2-yl)prop-2-ynyl.

A specific example of a R² moiety for the practice of this invention is hydrogen.

A specific example of a R³ moiety for the practice of this invention is ((phenyl)carbonyl)oxy.

A specific example of a R⁹ moiety for the practice of this invention is hydrogen.

Specific examples of R⁵ moieties for the practice of this invention are hydrogen and methyl.

A specific example of a R⁶ moiety for the practice of this invention is hydrogen.

A specific example of a R⁷ moiety for the practice of this invention is =0.

A specific examples of R⁸ and R⁹ moieties for the practice of this invention is R⁸ and R⁹ together are =0.

A specific example of a R¹⁰ moiety for the practice of this invention is hydrogen.

A specific example of a X¹ moiety for the practice of this invention is hydrogen.

These specific moieties may combine to provide a fifth embodiment of this invention, which embodiment is directed to compounds having formula (I) or formula (II), and pharmaceutically acceptable salts thereof, in which R¹ is alkyl, -(CH₂)alkynyl, or -(CH₂)alkynyl substituted with thienyl, in which the thienyl is substituted with pyridyl; R² is hydrogen; R³ is ((phenyl) carbonyl) oxy; R⁴ is hydrogen; R⁵ is hydrogen or alkyl; R⁶ is hydrogen; R⁷ is =O; R⁸ and R⁹ together are =O; R¹⁰ is hydrogen; and X¹ is hydrogen;
compounds having formula (I) or formula (II), and pharmaceutically acceptable salts thereof, in which R¹ is methyl, prop-2-ynyl or prop-2-ynyl substituted with thienyl, in which the thienyl is substituted with pyridyl; R² is hydrogen; R³ is ((phenyl)carbonyl)oxy; R⁴ is hydrogen; R⁵ is hydrogen or methyl; R⁶ is hydrogen; R⁷ is. =O; R⁸ and R⁹ together are =O; R¹⁰ is hydrogen; and x¹ is hydrogen;
compounds having formula (I) or formula (II), and pharmaceutically acceptable salts thereof, in which R¹ is methyl; R² is hydrogen; R³ is ((phenyl)carbonyl)oxy; R⁴ is hydrogen; R⁵ is hydrogen or methyl; R⁶ is hydrogen; R⁷ is =O; R⁸ and R⁹ together are =O; R¹⁰ is hydrogen; and X¹ is hydrogen;
compounds having-formula (I) or formula (II), and pharmaceutically acceptable salts thereof, in which R¹ is prop-2-ynyl; R² is hydrogen; R³ is ((phenyl) carbonyl) oxy; R⁴ is hydrogen; R⁵ is hydrogen or methyl; R⁶ is hydrogen; R⁷ is =O; R⁸ and R⁹ together are =O; R¹⁰ is hydrogen; and X¹ is hydrogen;
compounds having formula (I) or formula (II), and pharmaceutically acceptable salts thereof, in which R¹ is. 3-(5-pyridin-2-ylthien-2-yl)prop-2-ynyl; R² is hydrogen; R³ is ((phenyl)carbonyl)oxy; R⁴ is hydrogen; R⁵ is hydrogen or methyl; R⁶ is hydrogen; R⁷ is =O; R⁹ and R⁹ together are =O; R¹⁰ is hydrogen; and X¹ is hydrogen; and
compounds, and pharmaceutically acceptable salts thereof, which are
(2aR, 4aS, 6R, 8S, 9R, 10R, 12R, 15R, 15aS, 15bS) -15-ethyl-3, 4a, 6, 8, 10, 12, 15a-heptamethyl-2, 5, 11,13-tetraoxo-8-((3-(5-(pyridin-2-yl)thien-2-yl)prop-2-ynyl)oxy)hexadecahydro-2H-1,14-dioxa-3-azacyclotetradeca(1,2,3-cd)pentalen-9-yl 3,4,6-trideoxy-3-(dimethylamino)-β-D-xylo-hexopyranoside;
(2aR, 4aS, 6R, 8S, 9R, 10R, 12R, 15R, 15aS, 15bS)-15-ethyl-8-methoxy-3, 4a, 6, 8, 10, 12, 15a-heptamethyl-2, 5, 11, 13-tetraoxohexadecahydro-2H-1,14-dioxa-3-azacyclotetradeca-(1,2,3-cd)pentalen-9-yl 3, 4, 6-trideoxy-3-(dimethylamino)-β-D-xylo-hexopyranoside;
(2aS, 4aR, 6R, 8S, 9R, 10R, 12R, 15R, 15aS, 15bR)-15-ethyl-8-methoxy-3,4a,6,8,10,12,15a-heptamethyl-2,5,11,13-tetraoxohexadecahydro-2H-1,14-dioxa-3-azacyclotetradeca-(1,2,3-cd)pentalen-9-yl 3,4,6-trideoxy-3-(dimethylamino)-β-D-xylo-hexopyranoside;
(2aR, 4aS, 6R, 8S, 9R, 10S, 11S, 12R, 15R, 15aS, 15bS)-15-ethyl-3,4a,6,8,10,12,15a-heptamethyl-2,5,13-trioxo-8-(prop-2-ynyloxy)-9-((3,4,6-trideoxy-3-(dimethylamino)-β-D-xylo-hexopyranosyl)oxy)hexadecahydro-2H-1,14-dioxa-3-azacyclotetradeca(1,2,3-cd)pentalen-11-yl 4-O-benzoyl-2,6-dideoxy-3-C-methyl-3-O-methyl-α-L-ribo-hexopyranoside;
(2aR, 4aS, 6R, 8S, 9R,10R, 12R, 15A, 15aS, 15bS)-15-ethyl-3,4a,6,8,10,12,15a-heptamethyl-2,5,11,13-tetraoxo-8-(prop-2 - ynyloxy)-hexadecahydro-2H-1,14-dioxa-3-azacyclotetradeca-(1,2,3-cd)pentalen-9-yl 3,4,6-trideoxy-3-(dimethylamino)-β-D-xylo-hexopyranoside; or
(2aR, 4aS, 6R, 8S, 9R, 10R, 12R, 15R, 15aS, 15bS) -15-ethyl-8-methoxy-4a,6,8,10,12,15a-hexamethyl-2,5,11,13-tetraoxo hexadecahydro-2H-1,14-dioxa-3-azacyclotetradeca (1,2,3-cd)pentalen-9-yl 3,4,6-trideoxy-3-(dimethylamino)-β-D-xylo-hexopyranoside.

Compounds of this invention contain asymmetrically substituted carbon atoms in the R or S configuration, in which the terms "R" and "S" are as defined by the IUPAC 1974 Recommendations for Section E, Fundamental . Stereochemistry, Pure Appl. Chem. (1976) 45, 13-10. Compounds having asymmetrically substituted carbon atoms with equal amounts of R and S configurations are racemic at those carbon atoms. Atoms with an excess of one configuration over the other are assigned the configuration which is present in the higher amount, preferably an excess of about 85%-90%, more preferably an excess of about 95%-99%, and still more preferably an excess greater than about 99%. Accordingly, this invention is meant to embrace all stereoisomers of the compounds including racemic mixtures, enantiomers, mixtures of enantiomers, diastereomers, and mixtures of diastereomers.

Individual stereoisomers of the compounds may be prepared by any one of a number of methods within the knowledge of the ordinarily skilled practioner. These methods include stereospecific synthesis, chromatographic separation of diastereomers, chromatographic resolution of enantiomers, enzymatic resolution, and conversion of enantiomers in an enantiomeric mixture to diastereomers and chromatographically separating the diastereomers and regeneration of the individual enantiomers.

Stereospecific synthesis involves the use of appropriate chiral starting materials and synthetic reactions which do not cause racemization or inversion of stereochemistry at the chiral centers.

Diastereomeric mixtures of compounds resulting from a synthetic reaction can be separated by chromatographic techniques which are well-known to the ordinarily skilled practioner.

Chromatographic resolution of enantiomers can be accomplished on chiral commercially available chromatography resins. In practice, the racemate is placed in solution and loaded onto the column containing a chiral stationary phase. The enantiomers are then separated by high performance liquid chromatography.

Enzymes, such as esterases, phosphatases and lipases, may be useful for resolution of derivatives of the enantiomers in an enantiomeric mixture. For example, an ester derivative of a carboxyl group of the compounds to be separated can be prepared. Certain enzymes will selectively hydrolyze only one of the enantiomers in the mixture. Then the resulting enantiomerically pure acid can be separated from the unhydrolyzed ester.

Resolution of enantiomers may also be accomplished by converting the enantiomers in the mixture to diastereomers by reacting of the former and chiral auxiliaries. The resulting diastereomers can then be separated by column chromatography. This technique is especially useful when the compounds to be separated contain a carboxyl, amino or hydroxyl group that will form a salt or covalent bond with the chiral auxiliary. Chirally pure amino acids, organic carboxylic acids or organosulfonic acids are especially useful as chiral auxiliaries. Once the diastereomers have been separated by chromatography, the individual enantiomers can be regenerated. Frequently, the chiral auxiliary can be recovered and reused.

Compounds of this invention may also contain carbon-carbon double bonds in the Z or E configuration, in which the term "Z" represents the larger two substituents on the same side of a carbon-carbon double bond and the term "E" represents the larger two substituents on opposite sides of a carbon-carbon double bond. The compounds may also exist as an equilibrium mixture of Z or E configurations.

Compounds of this invention may exist as acid addition salts, basic addition salts, or zwitterions. Salts of the compounds are prepared during their isolation or following their purification. Acid addition salts of the compounds are those derived from the reaction of the compounds with an acid. For example, the acetate, adipate, alginate, bicarbonate, citrate, aspartate, benzoate, benzenesulfonate, bisulfate, butyrate, camphorate, camphorsufonate, digluconate, formate, fumarate, glycerophosphate, glutamate, hemisulfate, heptanoate, hexanoate, hydrochloride, hydrobromide, hydroiodide, lactobionate, lactate, maleate, mesitylenesulfonate, methanesulfonate, naphthylenesulfonate, nicotinate, oxalate, pamoate, pectinate, persulfate, phosphate, picrate, propionate, succinate, tartrate, thiocyanate, trichloroacetic, trifluoroacetic, para-toluenesulfonate, and undecanoate, salts of the compounds and prodrugs thereof are embraced by this invention. When the compounds contain carboxylic acids, basic addition salts may be prepared therefrom by reaction with a base such as the hydroxide, carbonate, and bicarbonate of cations such as lithium, sodium, potassium, calcium, and magnesium.

Compounds of this invention may be administered with or without an excipient. Excipients include encapsulating materials or formulation additives such as absorption accelerators, antioxidants, binders, buffers, coating agents, coloring agents, diluents, disintegrating agents, emulsifiers, extenders, fillers, flavoring agents, humectants, lubricants, perfumes, preservatives, propellants, releasing agents, sterilizing agents, sweeteners, solubilizers, wetting agents, and mixtures thereof. Excipients for orally administered compounds in solid dosage forms include agar, alginic acid, aluminum hydroxide, benzyl alcohol, benzyl benzoate, 1,3-butylene glycol, castor oil, cellulose, cellulose acetate, cocoa butter, corn starch, corn oil, cottonseed oil, ethanol, ethyl acetate, ethyl carbonate, ethyl cellulose, ethyl laureate, ethyl oleate, gelatin, germ oil, glucose, glycerol, groundnut oil, isopropanol, isotonic saline, lactose, magnesium hydroxide, magnesium stearate, malt, olive oil, peanut oil, potassium phosphate salts, potato starch, propylene glycol, Ringer's solution, talc, tragacanth, water, safflower oil, sesame oil, sodium carboxymethyl cellulose, sodium lauryl sulfate, sodiumphosphate salts, soybean oil, sucrose, tetrahydrofurfuryl alcohol, and mixtures. Excipients for ophthalmically and orally administered compounds in liquid dosage forms include benzyl alcohol, benzyl benzoate, 1,3-butylene glycol, castor oil, corn oil, cottonseed oil, ethanol, ethyl acetate, ethyl carbonate, fatty acid esters of sorbitan, germ oil, groundnut oil, glycerol, isopropanol, olive oil, polyethylene glycols, propylene glycol, sesame oil, tetrahydrofurfuryl alcohol, water, and mixtures thereof. Excipients for osmotically administered compounds include chlorofluorohydrocarbons, ethanol, isopropanol, water, and mixtures thereof. Excipients for parenterally administered compounds include 1,3-butanediol, castor oil, corn oil, cottonseed oil, germ oil, groundnut oil, liposomes, oleic acid, olive oil, peanut oil, Ringer's solution, safflower oil, sesame oil, soybean oil, U.S.P. or isotonic sodium chloride solution, water, and mixtures thereof. Excipients for rectally and vaginally administered compounds include cocoa butter, polyethylene glycol, wax, and mixtures thereof.

Compounds of this invention may be administered orally, ophthalmically, osmotically, parenterally (subcutaneously, intramuscularly, intrasternally, intravenously), rectally, topically, transdermally, and vaginally. Orally administered compounds in solid dosage forms may be administered as capsules, dragees, granules, pills, powders, and tablets. Ophthalmically and orally administered compounds in liquid dosage forms may be administered as elixirs, emulsions, microemulsions, solutions, suspensions, and syrups. Osmotically and topically administered compounds may be administered as creams, gels, inhalants, lotions, ointments, pastes, powders, solutions, and sprays. Parenterally administered compounds may be administered as aqueous or oleaginous solutions or aqueous or oleaginous and suspensions, in which suspensions comprise crystalline, amorphous, or otherwise insoluble forms of the compounds. Rectally and vaginally administered compounds may be administered as creams, gels, lotions, ointments, and pastes.

Therapeutically effective amounts of compounds of this invention depend on the recepient of treatment, the disorder being treated and the severity of the disorder, the composition comprising the compounds, the time of administration, the route of administration, the duration of treatment, the potency of the compounds, and the rate of excretion of the compounds. The daily therapeutically effective amount of the compounds administered to a patient in single or divided doses range from about 0.1 to about 200 mg/kg body weight, preferably from about 0.25 to about 100 mg/kg body weight. Single dose compositions contain these amounts of the compounds or combinations of submultiples thereof.

To determine antibacterial activity of the compounds of this invention, twelve petri dishes, each containing successive aqueous dilutions of test compounds in sterilized Brain Heart Infusion agar (Difco 0418-01-5) (10 mL), were inoculated with 1:100 dilutions of the representative microorganisms in TABLE 1 using a Steers replicator block (or 1:10 dilutions for slow-growing Streptococcus strains), co-incubated at 35-37°C for 20-24 hours with a plate with a control plate having no compound, and inspected visually to provide the minimum inhibitory concentration (MIC), in µg/mL, by which is meant the lowest concentration of the test compound which yielded no growth, a slight haze, or sparsely isolated colonies on the inoculums spot as compared to growth in the control plate.

**TABLE 1**

| Microorganism | Code |
|---|---|
| Staphylococcus aureus NCTC10649M | AA |
| Staphylococcus aureus A5177 | BB |
| Staphylococcus aureus PIU 2043 | CC |
| Staphylococcus aureus 1775 | DD |
| Streptococcus pyrogenes EES61 | EE |
| Streptococcus pyrogenes 930 | FF |
| Streptococcus pyrogenes PIU 2548 | GG |
| Streptococcus pneumoniae ATCC 6303 | HH |
| Streptococcus pneumoniae 5979 | JJ |
| Streptococcus pneumoniae 5649 | KK |
| Enterococcus faecalis PIU 1967 | LL |
| Enterococcus faecium GYR 1632 | MM |
| Moraxella catarrhalis 2604 | NN |
| Haemophilus influenzae GYR 1435 | PP |
| Escherichia coli JUHL | QQ |

The ability of the compounds to inhibit bacterial growth was superior to the control and in the range of about 0.5µg/mL to greater than about 128µg/mL against the microorganisms listed in TABLE 1. These data demonstrate the usefulness of the compounds as antibacterials.

It is meant to be understood that certain metabolites of compounds of this invention, which metabolites are produced by in vitro or in vivo metabolic processes, would also be useful as antibacterials and are meant to be embraced by this invention.

It is still also meant to be understood that certain precursor compounds, which precursor compounds may be metabolized in vitro or in vivo to form compounds of this invention, are meant to be embraced by this invention.

Compounds of this invention may also be prepared by synthetic chemical processes, examples of which synthetic chemical processes, and intermediates employed in the processes, are shown hereinbelow. It is meant to be understood that the order of the steps in the processes may be varied, reagents, solvents, and reaction conditions may be substituted for those specifically mentioned, and vulnerable moieties may be protected and deprotected, as necessary, during the process.

Abbreviations used herein are DMF for N,N-dimethylformamide; THF for tetrahydrofuran.

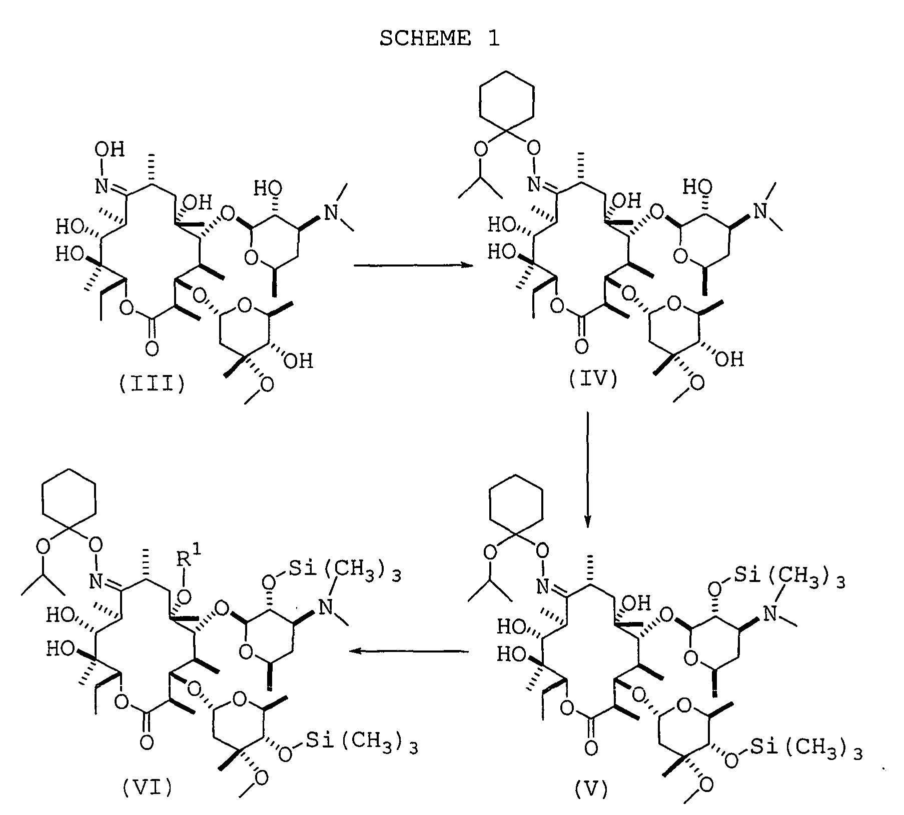

The compound having formula (III) may be prepared from erythromycin A as described in U.S. 5,274,085, column 3, lines 41-48 and 5,808,017, column 4, lines 37-53.

The compound having formula (III) may be converted to the compound having formula (IV) as described in U.S. 4,990,602, column 23, lines 11-19.

The compound having formula (IV) may be converted to the compound having formula (V) as described in U.S. 4,990,602, column 23, lines 34-42.

The compound having formula (V) may be converted to compounds having formula (VI) by reacting the former, a compound having formula

X²-R¹,

in which X² is -Cl or -Br, and a first base.

Examples of compounds having formula X²-R¹ include compounds having formula X²-R¹¹, X²-C(O)OR¹¹, X²-C (O) NH₂, X²-C(O)NHR¹², and X²-C(O)NR¹²R¹³.

Examples of compounds having formula X²-R¹¹ include bromomethane, 3-bromoprop-1-ene, 3-bromoprop-1-yne, benzyl bromide, 2-fluoroethyl bromide, 4-nitrobenzyl bromide, 4-chlorobenzyl bromide, 4-methoxybenzyl bromide, (3-bromoprop-1-enyl)benzene, 1-bromobut-2-ene, 2-(5-(3-bromoprop-1-ynyl)thien-2-yl)pyridine, 1-bromopent-2-ene, 2-(3-bromoprop-1-enyl)naphthalene, 5-(3-bromoprop-1-ynyl)-2-thien-2-ylpyridine, 2-(3-bromoprop-1-ynyl)pyridine, 3-((1E)-3-bromoprop-1-enyl)quinoline, 2-(5-(3-bromoprop-1-ynyl)isoxazol-3-yl)pyridine, and 2-(5-(3-bromoprop-1-ynyl)thien-2-yl)pyrimidine.

Examples of compounds having formula X²-C(O)OR¹¹ include ethyl chloroformate, methyl chloroformate, phenyl chloroformate, propargyl chloroformate, allyl chloroformate, 2-bromoethyl chloroformate, 1-chloroethyl chloroformate, 3-chloropropyl formate, 4-chlorobutyl formate, 3-butenyl chloroformate, 2-methoxyphenyl chloroformate, para-toluene chloroformate, and 4-methoxyphenyl chloroformate.

Examples of compounds having formula formula X²-C(O)NH₂ are carbamic chloride and carbamic bromide.

Examples of compounds having formula X²-C(O)NHR¹² include 4-chlorophenylcarbamic chloride, 5-bromo-1,1'-biphenyl-2-ylcarbamic chloride, quinolin-8-ylcarbamic chloride, 2-methoxyphenylcarbamic chloride, methylcarbamic chloride, cyclohexylcarbamic chloride, 2-(dimethylamino)-4-methoxyphenylcarbamic chloride, prop-2-ynylcarbamic chloride, 3-(5-(pyridin-2-yl)thien-2-yl)prop-2-ynylcarbamic chloride, and 2,6-dimethylphenylcarbamic chloride.

Examples of compounds having formula X²-C(O)NR¹²R¹³ include dimethylcarbamic chloride, diethylcarbamic chloride, diisopropylcarbamic chloride, diallylcarbamic chloride, 4-ethoxyphenyl(pyridin-2-yl)carbamic chloride, methyl(phenyl)carbamic chloride, methyl(vinyl)carbamic chloride, diphenylcarbamic chloride, ethyl(3-(trifluoromethyl)-1,2,4-thiadiazol-5-yl)carbamic chloride, and 2-chloroprop-2-enyl(propyl)carbamic chloride.

Examples of first bases include pyridine, triethylamine, diisopropylethylamine, 4-(N,N-dimethylamino)pyridine, sodium hydroxide, potassium hydroxide, potassium tert-butoxide, sodium carbonate, sodium bicarbonate, cesium hydroxide, tetramethylammonium hydroxide, sodium hydride, potassium hydride, potassium isopropoxide, potassium isobutoxide, and mixtures thereof.

The reaction is typically conducted over about 0.5 hours to about 8 hours, at about -15°C to about 50°C, in solvents such as tetrahydrofuran, diethylether, ethyl acetate, acetone, N,N-dimethylformamide, dimethylsulfoxide, diethylsulfoxide, 1,2-dimethoxyethane, dichloromethane, chloroform, and mixtures thereof.

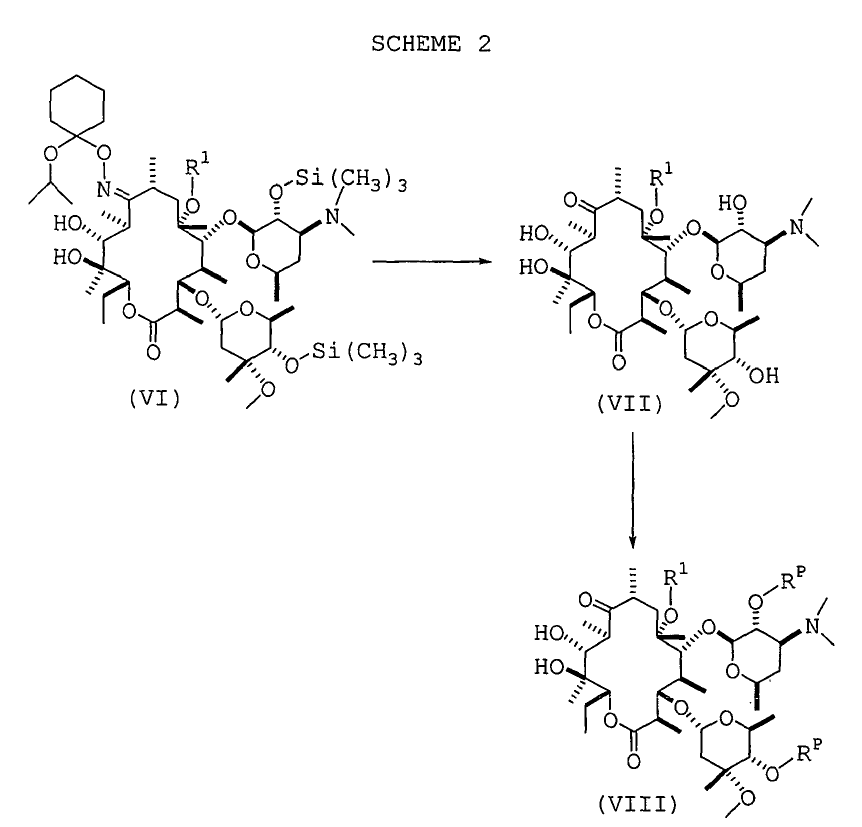

Compounds having formula (VI) may be converted to compounds having formula (VII) as described in U.S. 4,672,109, column 19-20, lines 54-64 and 1-2, respectively and U.S. 5,808,017, column 4, lines 21-29.

Compounds having formula (VII) may be converted to compounds having formula (VIII), in which R^{P} is acetyl (CH₃C(O)-), or benzoyl (C₆H₅C(O)-), by reacting the former, a hydroxyl protecting group precursor and a second base, with or without 4-(N,N-dimethylamino)pyridine.

Examples of hydroxyl protecting group precursors include acetic anhydride and benzoic anhydride.

Examples of second bases include triethylamine, diisopropylethylamine, and pyridine.

The reaction is typically conducted over about 1 hour to about 24 hours, at about 25°C to about 75°C, in solvents such as tetrahydrofuran, dimethylsulfoxide, acetonitrile, dichloromethane, chloroform, N,N-dimethylformamide, and mixtures thereof.

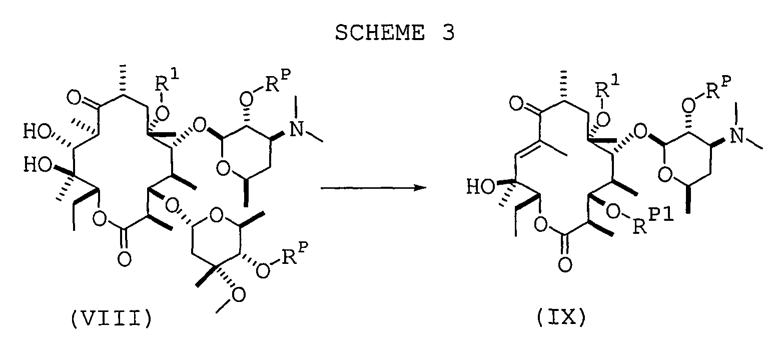

Compounds having formula (VIII) may be converted to compounds having formula (IX), in which R^{P1} is trialkylsilyl, by (a) protecting the 11,12-diol using the same reagents and under the same conditions as described in J.Org. Chem., Vol. 53, No. 10, 1988, p.2344, (b) removing the cladinose moiety from the product obtained from step (a) using the same reagents and under the same conditions described for the conversion of the compounds having formula (I)-a to the compounds having formula (II)-a in SCHEME 9, (c) silylating the product obtained from step (b) using the same reagents and under the same conditions described for the conversion of the compounds having formula (IV) to the compounds having formula (V) in SCHEME 1, and (d) dehydrating the product from step (c) using the same reagents and under the same conditions described in J.Org. Chem., Vol. 53, No. 10, 1988, p.2344.

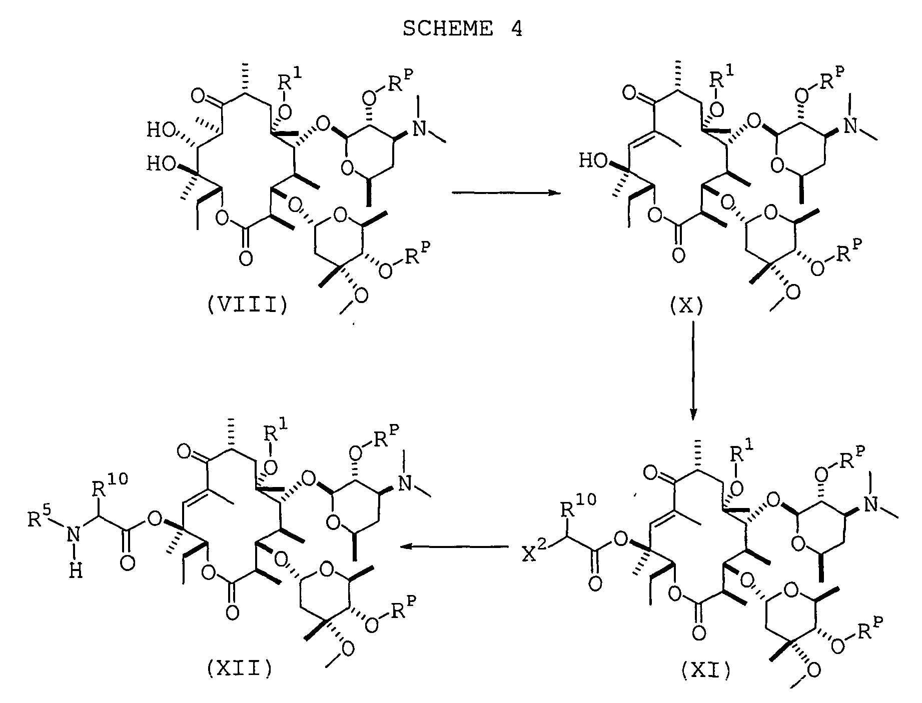

Compounds having formula (VIII) may be converted to compounds having formula (X) by (a) reacting the former and an activating agent, with or without the second base, and with or without 4-(N,N-dimethylamino)pyridine, and (b) reacting the product of step (a) and a third base.

Examples of activating agents include methanesulfonyl chloride, methanesulfonic anhydride, para-toluenesulfonyl chloride, and acetic anhydride.

Examples of third bases include sodium bis(trimethylsilyl)amide, potassium bis(trimethylsilyl)amide, lithium diisopropylamide, lithium tetramethylpiperidide, tetramethylguanidine, 1,8-diazabicyclo(5.4.0)undec-7-ene, and mixtures thereof.

Step (a) is typically conducted over about 1 hour to about 24 hours, at about -10°C to about 40°C, in solvents such as pyridine, tetrahydrofuran, ether, dichloromethane, chloroform, dimethylsulfoxide, N,N-dimethylformamide, acetonitrile, and mixtures thereof.

Step (b) is typically conducted over about 1 hour to about 3 days, at about -78°C to about 80°C, in solvents such as acetone, tetrahydrofuran, N,N-dimethylformamide, dioxane, 1,2-dimethoxyethane, acetonitrile, and mixtures thereof.

Compounds having formula (X) may be converted to compounds having formula (XI) by reacting the former, a compound having formula (X²CHR¹⁰ CO)₂O, and the second base, with or without 4-(N,N-dimethylamino)pyridine.

Examples of compounds having formula (X²CHR¹⁰CO)₂O include chloroacetic anhydride, 2-chloropropanoic anhydride, and 2-chlorobutanoic anhydride, bis(1-chlorobut-3-enyl) carbonate, and bis(1-chlorobut-3-ynyl) carbonate.

The reaction is typically conducted over about 1 hour to about 72 hours, at about -10°C to about 35°C, in solvents such as dichloromethane, tetrahydrofuran, acetonitrile, chloroform, N,N-dimethylformamide, 1,2-dimethoxyethane, and mixtures thereof.

Compounds having formula (XI) may be converted to compounds having formula (XII) by reacting the former and a compound having formula R⁵NH₂.

Examples of compounds having formula R⁵NH₂ include allylamine, benzylamine, 2,4-dimethoxybenzylamine, ethylamine, 4-methoxybenzylamine, methylamine, propylamine, propargylamine, propylamine, 3-(5-pyridin-2-yl-thiophen-2-yl)allylamine, 3-(5-pyridin-2-yl-thiophen-2-yl)propargylamine, and (2E)-3-quinolin-3-ylprop-2-en-1-amine.

The reaction is typically conducted over about 1 hour to about 72 hours, at about -10°C to about 50°C, in the compound having R⁵NH₂ itself or in solvents such as N,N-dimethylformamide, pyridine, dichloromethane, chloroform, tetrahydrofuran, and mixtures thereof.

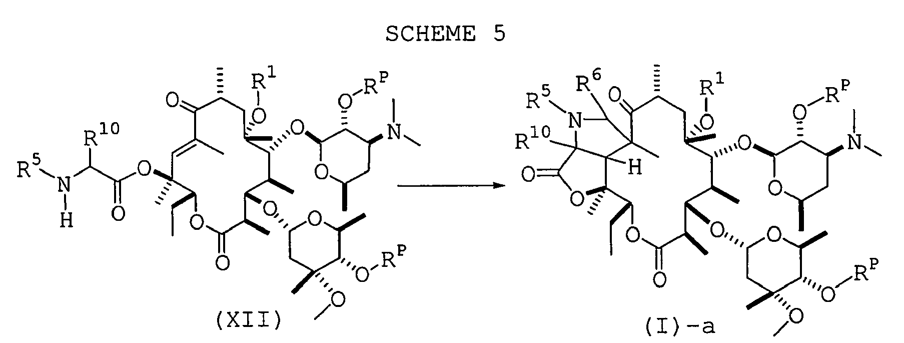

Compounds having formula (XII) may be converted to compounds having formula (I)-a by reacting the former, a compound having formula R⁶CHO or an acetal thereof, and a first acid.

Examples of compounds having formula R⁶CHO include acetaldehyde, acrolein, benzaldehyde, formaldehyde, 4-pentenaldehyde, and propionaldehyde.

Examples of first acids include hydrochloric acid, para-toluenesulfonic acid, acetic acid, formic acid, boron trifluoride, and aluminum chloride.

The reaction is typically conducted at about 25°C to about 150°C, over about 1 hour to about 10 days, in solvents such as toluene, benzene, xylene, and mixtures thereof.

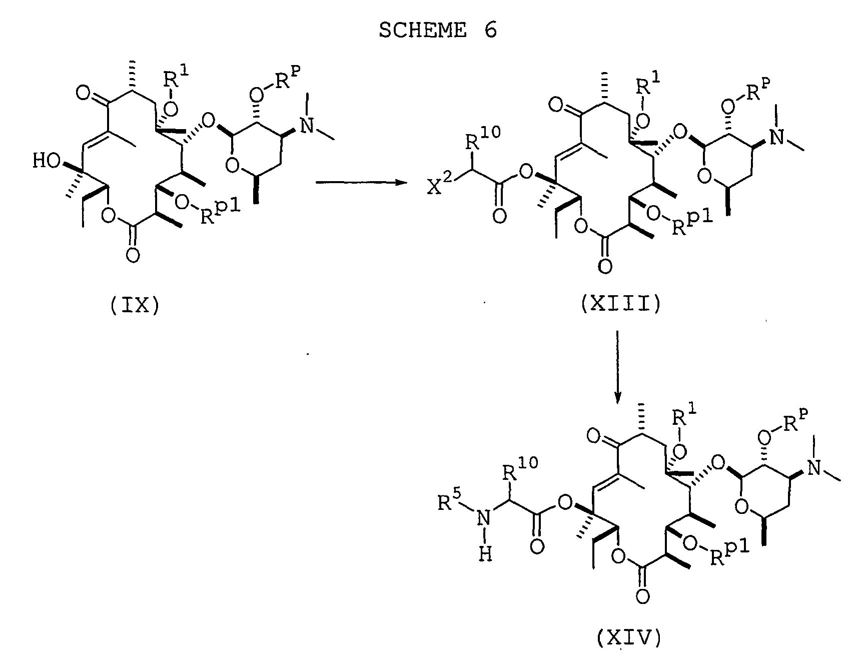

Compounds having formula (IX), in which R^{P1} is trimethylsilyl or triethylsilyl, may be converted to compounds having formula (XIV) using the same reagents and under the same conditions described for the conversion of compounds having formula (X) to compounds having formula (XII) in SCHEME 4.

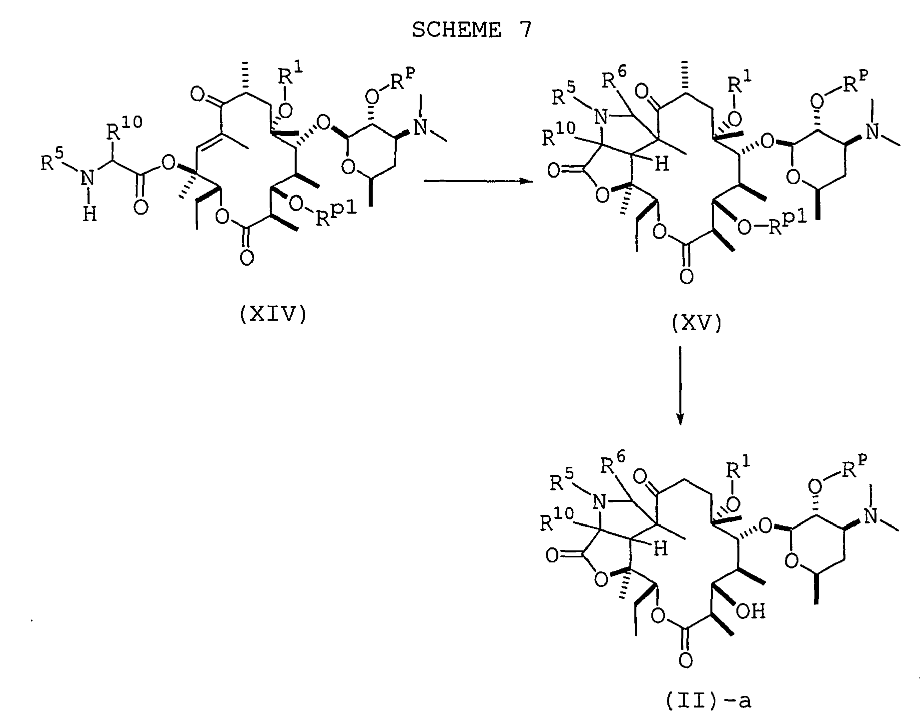

Compounds having formula (XIV) may be converted to compounds having formula (XV) using the same reagents and under the same conditions described for the conversion of compounds having formula (XII) to compounds having formula (I)-a in SCHEME 5.

Compounds having formula (XV) may be converted to compounds having formula (II)-a by reacting the former and a fluoride-donating agent.

Examples of fluoride-donating agents include tetrabutylammonium fluoride, tetrabutylammonium chloride/potassium fluoride monohydrate, HF·pyridine, hydrogen fluoride, and ammonium fluoride.

The reaction is typically conducted at about 25°C to about 100°C, over about 1 hour to about 48 hours, in solvents such as benzene, toluene, tetrahydrofuran, water, acetone, and mixtures thereof.

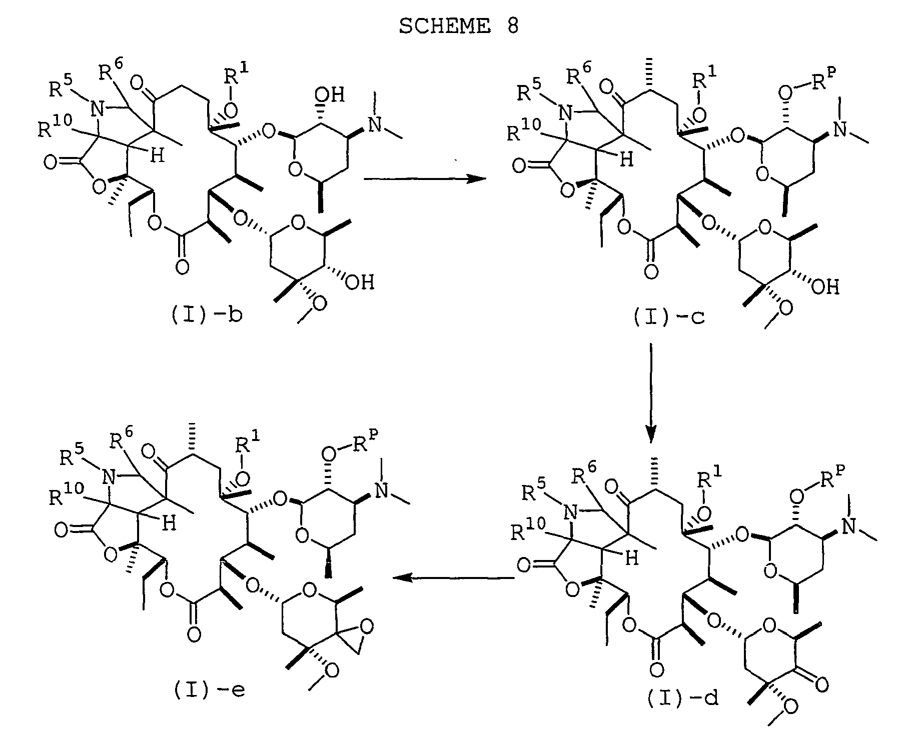

Compounds having formula (I)-b may be converted to compounds having formula (I)-c using the same reagents and under the same conditions described for the conversion of compounds having formula (VII) to compounds having formula (VIII) in SCHEME 2 except using only one equivalent of the hydroxyl protecting group precursor.

Compounds having formula (I)-c may be converted to compounds having formula (I)-d by reacting the former and an oxidant, with or without the second base.

Examples of oxidants include triacetoxy periodinane, N-chlorosuccinimide·dimethyl sulfide, dicyclohexyl carbodiimide·dimethyl sulfoxide·pyridinium trifluoroacetate, oxalyl chloride·dimethylsulfoxide, and sulfur trioxide·pyridine·dimethylsulfoxide.

The reaction is typically conducted at about -20°C to about 35°C, over about 1 hour to about 72 hours, in solvents such as water, dichloromethane, acetonitrile, chloroform, tetrahydrofuran, and mixtures thereof.

Compounds having formula (I)-d may be converted to compounds having formula (I)-e by reacting the former and a sulfur ylide.

Examples of sulfur ylides include dimethyloxosulfonium methylide and dimethylsulfonium methylide.

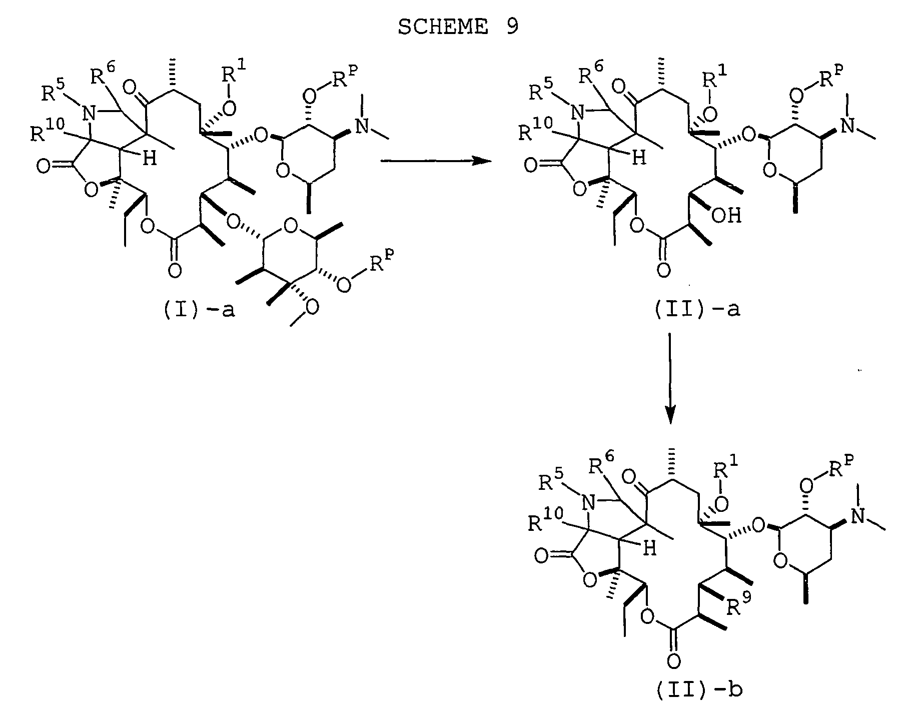

Compounds having formula (I)-a may be converted to compounds having formula (II)-a by reacting the former and a second acid.

Examples of second acids include hydrochloric acid, sulfuric acid, perchloric acid, chloroacetic acid, dichloroacetic acid, and trifluoroacetic acid.

The reaction is typically conducted at about -10°C to about 70°C, over about 1 hour to about 72 hours, in solvents such as dichloromethane, tetrahydrofuran, methanol, ethanol, isopropanol, butanol, and mixtures thereof.

Compounds having formula (II)-a may be converted to compounds having formula (II)-b, in which R⁹ is other than -OH, by reacting the former and a compound having formula X²-R³² using the same reagents and under the same conditions described for the conversion of compounds having formula (V) to compounds having formula (VI) in SCHEME 1.

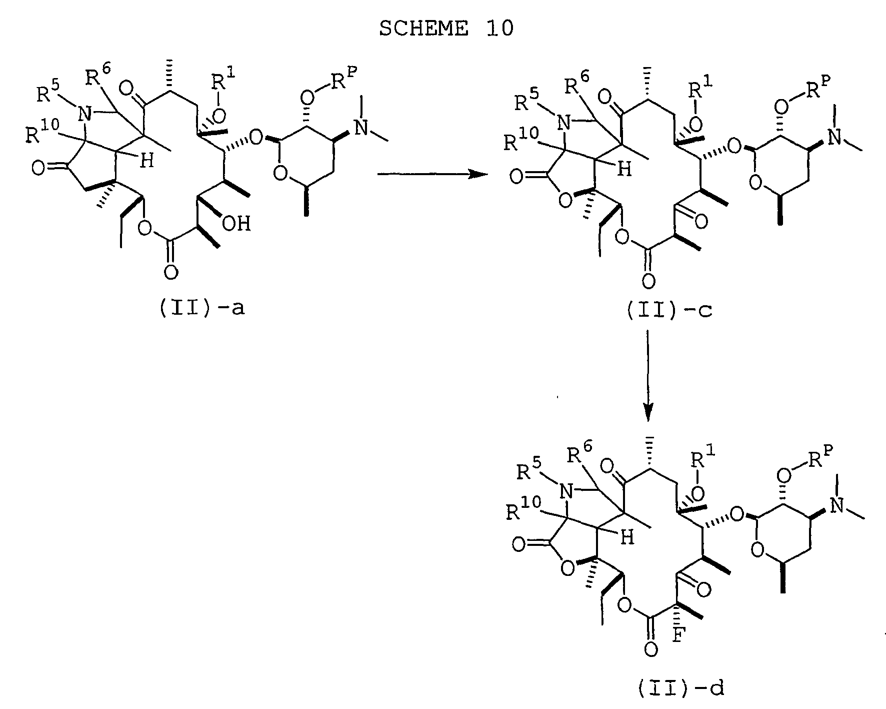

compounds having formula (II)-a may be converted to compounds having formula (II)-c using the same reagents and under the same conditions described for the conversion of compounds of formula (I)-c to compounds of formula (I)-d in SCHEME 8.

Compounds having formula (II)-c may be converted to compounds having formula (II)-d by reacting the former and a fluorinating agent, with or without a fourth base.

Examples of fluorinating agents used without the fourth base include 10% F₂ in formic acid,
3,5-dichloro-1-fluoropyridinium tetrafluoroborate,
3,5-dichloro-1-fluoropyridinium triflate, and
N-tetrafluoro-N-((trifluoromethyl)sulfonyl)methane-sulfonamide. Examples of fluorinating agents used with the fourth base include N-fluorobenzesulfonimide,
N-fluoro-N-methyl-para-toluenesulfonamide, N-fluoropyridinim triflate, 1-(chloromethyl)-4-fluoro-1,4-diazoniabicyclo(2.2.2)octane bis(tetrafluoroborate) (SELECTFLUOR^{™}), and
N-fluoroperfluoropiperidine.

Examples of fourth bases include sodium hydride, potassium hydride, trimethylamine, lithium bis(triethylsilyl)amide, sodium bis(trimethylsilyl)amide, and potassium bis(trimethylsilyl)amide.

Compounds having formula (I) or formula (II), in which R^{p} is acetyl or benzoyl, may be converted to compounds having formula (I) or formula (II), in which R² is hydrogen, by reacting the former and a deprotecting agent.

Examples of deprotecting agents include acids such as methanol, ethanol, acetic acid, and formic acid and bases such as lithium hydroxide, sodium hydroxide, potassium hydroxide, potassium carbonate, and ammonia.

The reaction is typically conducted at about 25°C to about 70°C, over about 1 hour to about 72 hours, in solvents such as water, methanol, ethanol, and mixtures thereof.

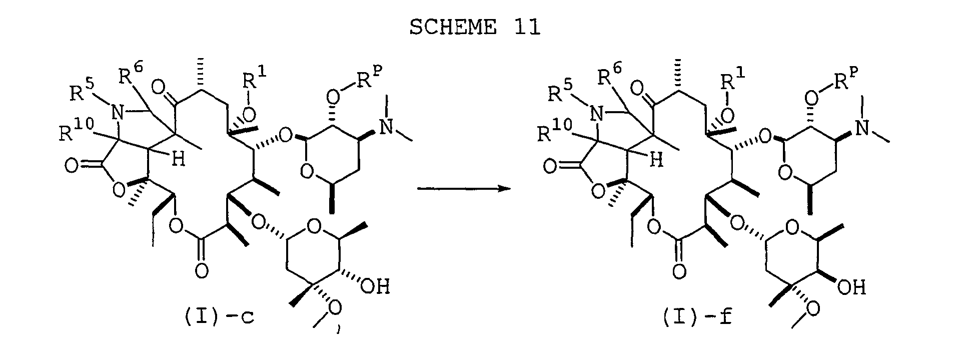

Compounds having formula (I)-c may be converted to compounds having formula (I)-f by (a) reacting the former, a third acid, a diazo compound, and a phosphine, and (b) reacting the product of step (a) and an alkali metal hydroxide.

Examples of third acids include benzoic acid and 4-nitrobenzoic acid.

Examples of diazo compounds include diethyl azodicarboxylate and diisopropyl azodicarboxylate.

Examples of phosphines include triphenyl phosphine and tributyl phosphine.

Examples of alkali metal hydroxides include lithium hydroxide, sodium hydroxide, and potassium hydroxide.

Step (a) is typically conducted over about 1 hour to about 8 hours, at about 0°C to about 85°C, in solvents such as tetrahydrofuran, dichloromethane, chloroform, benzene, toluene, xylene, and mixtures thereof.

Step (b) is typically conducted over about 1 hour to about 24 hours, at about 25°C to about 55°C, in solvents such as methanol, ethanol, isopropanol, and mixtures thereof.

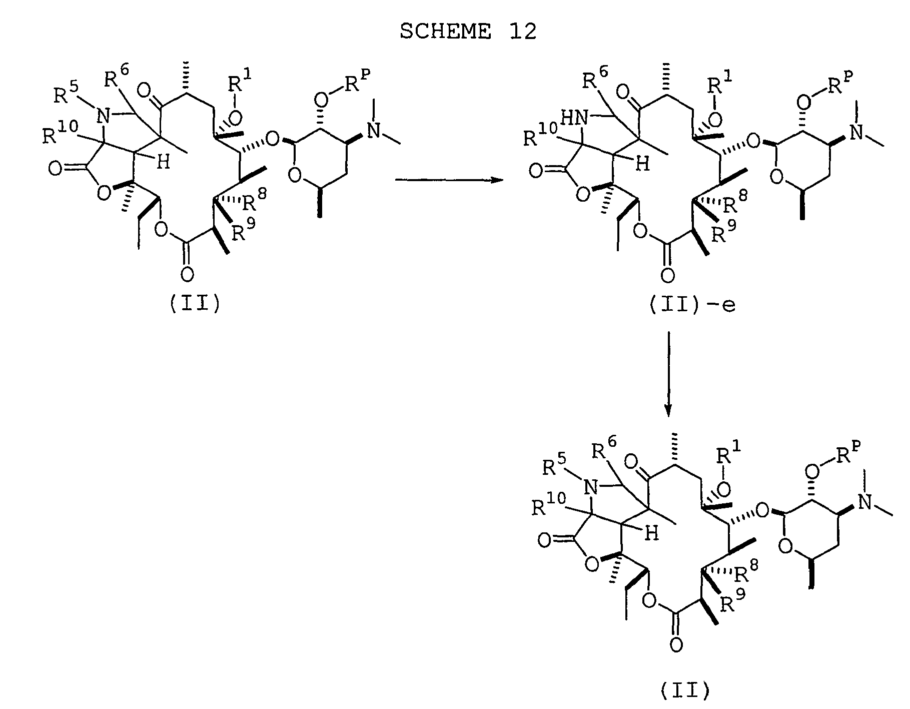

Compounds having formula (II), in which R⁵ is prop-2-enyl, may be converted to compounds having formula (II)-e by reacting the former, a palladium reagent, and a fourth acid.

The reaction is typically conducted at about 25°C to about 100°C, over about 2 hours to about 48 hours, in solvents such as toluene, tetrahydrofuran, dichloromethane, 1,2-dimethoxyethane, and mixtures thereof.

Examples of the palladium reagents include palladium on carbon, tetrakis palladium(0)(triphenylphosphine), and Pd(dibenzylidineacetone)diphenylphosphinobutane.

Examples of the fourth acids include methanesulfonic acid, thiobenzoic acid, and N,N-dimethylbarbituric acid.

Compounds having formula (II), in which R⁵ is 2,4-dimethoxybenzyl, may be converted to compounds having formula (II)-e by reacting the former and trifluoroacetic acid.

The reaction is typically conducted at about 25°C to about 75°C, over about 2 hours to about 48 hours, in solvents such as dichloromethane, chloroform, acetonitrile, tetrahydrofuran, and mixtures thereof.

Compounds having formula (II)-e may be converted to compounds having formula (II) by reacting the former and a compound having formula X²-R⁵ under the same conditions described for the conversion of compounds of formula (V) to compounds of formula (VI) in SCHEME 1.

Examples of compounds having formula X²-R⁵ include compounds having formula X²-R¹⁹, X²-C(O)OR¹⁹, X²-C (O) NH₂, X²-C (O) NHR²⁰, and X²-C (O) NR²⁰R²¹_{.}

Examples of compounds having formula X² -R¹⁹ include bromoinethaize, 3-bromoprop-1-ene, 3-bromoprop-1-yne, benzyl bromide, 2-fluoroethyl bromide, 4-nitrobenzyl bromide, 4-chlorobenzyl bromide, 4-methoxybenzyl bromide, (3-bromoprop-1-enyl)benzene, 1-bromobut-2-ene, 2-(5-(3-bromoprop-1-ynyl)thien-2-yl)pyridine, 1-bromopent-2-ene, 2-(3-bromoprop-1-enyl)naphthalene, 5-(3-bromoprop-1-ynyl)-2-thien-2-ylpyridine, 2-(3-bromoprop-1-ynyl)pyridine, 3-((lE)-3-bromoprop-1-enyl)quinoline, 2-(5-(3-bromoprop-1-ynyl)isoxazol-3-yl)pyridine, and 2-(5-(3-bromoprop-1-ynyl)thien-2-yl)pyrimidine.

Examples of compounds having formula X²-C(O)OR¹⁹ include ethyl chloroformate, methyl chloroformate, phenyl chloroformate, propargyl chloroformate, allyl chloroformate, 2-bromoethyl chloroformate, 1-chloroethyl chloroformate, 3-chloropropyl formate, 4-chlorobutyl formate, 3-butenyl chloroformate, 2-methoxyphenyl chloroformate, para-toluene chloroformate, and 4-methoxyphenyl chloroformate.

Examples of compounds having formula formula X²-C(O)NH₂ are carbamic chloride and carbamic bromide.

Examples of compounds having formula X²-C(O)NHR²⁰ include 4-chlorophenylcarbamic chloride, 5-bromo-1,1'-biphenyl -2-ylcarbamic chloride, quinolin-8-ylcarbamic chloride, 2-methoxyphenylcarbamic chloride, methylcarbamic chloride, cyclohexylcarbamic chloride, 2-(dimethylamino)-4-methoxyphenylcarbamic chloride, prop-2-ynylcarbamic chloride, 3-(5-(pyridin-2-yl)thien-2-yl)prop-2-ynylcarbamic chloride, and 2,6-dimethylphenylcarbamic chloride.

Examples of compounds having formula X²-C(O)NR²⁰R²¹ include dimethylcarbamic chloride, diethylcarbamic chloride, diisopropylcarbamic chloride, diallylcarbamic chloride, 4-ethoxyphenyl(pyridin-2-yl)carbamic chloride, methyl(phenyl)carbamic chloride, methyl(vinyl)carbamic chloride, diphenylcarbamic chloride, ethyl(3-(trifluoromethyl)-1,2,4-thiadiazol-5-yl)carbamic chloride, and 2-chloroprop-2-enyl(propyl)carbamic chloride.

Compounds having formula (II)-e may also be converted to compounds having formula (II), in which R⁵ is -R¹⁹, by reacting the former, a compound having formula R⁵CHO or the corresponding acetal, and a reducing agent, with or without the first acid.

Examples of reducing agents include sodium borohydride, sodium cyanoborohydride, sodium triacetoxyborohydride, zinc/hydrochloric acid, iron pentacarbonyl/alcoholic potassium hydroxide, borane·pyridine, and formic acid.

Examples of compounds having formula R⁵CHO include formaldehyde, acrolein, 4-pentenaldehyde, acetaldehyde, propionaldehyde, and benzaldehyde.

The reaction is typically conducted at about -10°C to about 150°C, over about 1 hour to about 10 days, in solvents such as tetrahydrofuran, dichloromethane, toluene, benzene, xylene, N,N-dimethylformamide, and mixtures thereof.

The following examples illustrate methods by which certain preferred first embodiments of the invention may be prepared.

### EXAMPLE 1

### compound having formula (VIII): R¹ is -CH₂C≡C-H; R^{P} is -C (O) (phenyl)

This example was prepared from erythromycin A (obtained from Abbott Laboratories) as described in SCHEME 1 and SCHEME 2. ¹³C NMR (CDCl₃) δ 219.9, 174.9, 166.1, 165.4, 133.4, 132.6, 130.8, 129.8, 129.6, 128.4, 128.2, 99.9, 95.9, 81.1, 80.4, 80.1, 78.8, 78.2, 76.6, 74.4, 73.8, 72.9, 72.6, 68.7, 67.5, 63.6, 51.6, 49.6, 45.2, 44.6, 40.9, 38.0, 37.8, 37.4, 35.4, 31.6, 26.9, 21.3, 21.1, 20.2, 18.5, 18.2, 16.2, 16.1, 12.3, 10.5, 9.5.

### EXAMPLE 2

### compound having formula (X): R¹ is -CH₂C=C-H, R^{P} is -C (O) (phenyl)

A solution of EXAMPLE 1 (2.605g) in pyridine (13 mL) at 10°C was treated with methansulfonic anhydride (1.2 g), stirred at 25°C for 20 hours, and concentrated. The concentrate was dissolved in dichloromethane (50 mL), washed with saturated aqueous NaHCO₃, and dried (Na₂SO₄), filtered, and concentrated. A solution of the concentrate and 1,8-diazabicyclo(5.4.0)undec-7-ene (0.60 mL) in acetone (15 mL) was stirred for 20 hours and concentrated; and the concentrate was flash chromatographed on silica gel with 15-40% acetone/hexane. ¹H NMR (CDCl₃) δ 8.06-7.98 (m, 4H), 7.63-7.38 (m, 6H), 6.48 (s, 1H), 5.05 (dd, J=10.5, 7.4Hz, 1H), 4.97-4.87 (m, 3H), 4.82 (d, J=7.2Hz, 1H), 4.51 (m, 1H), 4.23 (m, 2H), 3.94 (d, J=7.8Hz, 1H), 3.82 (m, 1H), 3.63 (d, J=6.6Hz, 1H), 3.48 (s, 3H), 3.34 (m, 1H), 2.93 (m, 1H), 2.75 (m, 1H), 2.58 (d, J=15.3Hz, 1H), 2.36 (t, J=2.1Hz, 1H), 2.31 (s, 6H), 2.00 (d, J=0.9Hz, 2H), 1.91-1.70 (m, 6H), 1.54 (s, 3H), 1.51 (m, 2H), 1.43 (m, 4H), 1.28-1.16 (m, 13H), 1.00 (d, J=6Hz, 3H), 0.86 (t, J=7.2Hz, 3H), 0.75 (d, J=7.5Hz, 3H).

### EXAMPLE 3A

### compound having formula (XI): R¹ is -CH₂C=C-H, R¹⁰ is hydrogen, R^{P} is -C (O) (phenyl)

A solution of EXAMPLE 2 (2.19g), triethylamine (300 µL), and 4-(N,N-dimethylamino)pyridine (20 mg) in dichloromethane (50 mL) at 0°C was treated with chloroacetic anhydride (840 mg), stirred at 25°C for 30 minutes, washed with saturated aqueous NaHCO₃, and dried (Na₂SO₄), filtered, and concentrated; and the concentrate was flash chromatographed on silica gel with 15-50% acetone/hexane. ¹H NMR (CDCl₃) δ 8.07-7.98 (m, 4H), 7.63-7.38 (m, 6H), 6.60 (s, 1H), 5.75 (dd, J=10.2, 3.3Hz, 1H), 5.03 (m, 2H), 4.94 (d, J=9.9Hz, 1H), 4.82 (d, J=7.8Hz, 1H), 4.51 (m, 1H), 4.22 (m, 2H), 3.96 (s, 2H), 3.81 (m, 2H), 3.62 (d, J=6.6Hz, 1H), 3.46 (s, 3H), 3.41 (m, 1H), 2.91 (m, 1H), 2.79 (m, 1H), 2.45 (d, J=14.7Hz, 1H), 2.38 (t, J=2.1Hz, 1H), 2.30 (s, 6H), 1.83 (s, 2H), 1.75 (m, 4H), 1.54 (s, 3H), 1.50 (m, 4H), 1.36 (s, 3H), 1.24 (m, 11H), 1.15 (d, J=6.3Hz, 3H), 1.00 (m, 2H), 0.84 (t, J=7.5Hz, 3H), 0.75 (d, J=7.5Hz, 3H).

### EXAMPLE 3B

### compound having formula (XII): R¹ is -CH₂C≡C-H, R⁵ is methyl, R¹⁰ is hydrogen, R^{P} is -C (O) (phenyl)

A solution of EXAMPLE 3A (2.38 g) and 2M methylamine in THF (3.5 mL) in DMF (20 mL) at 25°C was stirred for 18 hours, treated with more 2M methylamine in tetrahydrofuran (3 mL), stirred for 48 hours, and concentrated; and the concentrate was flash chromatographed on silica gel with 0.25% concentrated ammonium hydroxide/(95:5 dichloromethane/methanol). ¹³C NMR (CDCl₃) δ 205.8, 174.0, 169.8, 166.2, 165.2, 140.2, 137.2, 133.4, 132.5, 130.7, 129.8, 129.7, 128.4, 128.1, 100.5, 96.5, 81.9, 81.0, 80.4, 79.7, 79.1, 78.9, 76.6, 75.6, 73.4, 72.9, 72.3, 67.9, 63.6, 63.5, 51.9, 51.8, 49.7, 45.1, 40.8, 39.6, 39.1, 36.0, 35.6, 32.0, 23.6, 21.7, 21.25, 21.2, 19.0, 18.5, 17.2, 16.1, 13.0, 10.1.

### EXAMPLE 3C

### compound having formula (I)-a: R¹ is -CH₂C≡C-H, R⁵ is methyl, R¹⁰ is hydrogen, R^{P} is -C (O) (phenyl)

A solution of EXAMPLE 3B (1.57g), 37% aqueous formaldehyde (115 µL), and acetic acid (2 drops) in toluene (40 mL) was stirred for 30 minutes at 25°C and at 110°C for 1.5 hours under a Dean-Stark trap, and concentrated; and the concentrate was flash chromatographed on silica gel with 10-25% acetone/hexane. ¹³C NMR (CDCl₃) δ 217.4, 178.1, 176.8, 166.2, 165.3, 133.3, 132.6, 130.7, 129.9, 129.8, 129.6, 128.4, 128.2, 100.8, 95.4, 86.4, 83.0, 80.7, 79.6, 79.0, 78.5, 76.5, 74.9, 70.3, 72.2, 67.8, 67.2, 66.2, 63.7, 63.4, 56.5, 53.0, 52.0, 49.7, 45.1, 42.0, 41.3, 40.9, 39.9, 37.8, 35.0, 32.2, 23.9, 23.6, 21.5, 21.3, 21.2, 19.6, 18.4, 16.2, 15.2, 10.5, 9.5.

### EXAMPLE 3D

### (2aR, 4aS, 6R, 8S, 9R, 10S, 11S, 12R, 15R, 15aS, 15bS) -15-ethyl-3,4a,6,8,10,12,15a-heptamethyl-2,5,13-trioxo-8-(prop-2-ynyloxy)-9-((3,4,6-trideoxy-3-(dimethylamino)-β-D-xylohexopyranosyl)oxy)hexadecahydro-2H-1,14-dioxa-3-azacyclotetradeca(1,2,3-cd)pentalen-11-yl 4-O-benzoyl-2,6-dideoxy-3-C-methyl-3-O-methyl-α-L-ribo-hexopyranoside;

A solution of EXAMPLE 3C (60 mg) in methanol (10 mL) was refluxed for 24 hours and concentrated; and the concentrate was flash chromatographed on silica gel with 84:15:1 dichloromethane/methanol/concentrated ammonium hydroxide. ¹³C NMR (CDCl₃) δ 217.0, 178.2, 176.9, 166.1, 133.3, 130.0, 129.6, 128.3, 102.8, 95.4, 86.5, 83.0, 80.7, 79.6, 79.1, 78.8, 76.7, 75.0, 72.8, 71.0, 68.1, 67.2, 66.2, 65.4, 63.4, 56.6, 53.0, 52.1, 49.6, 45.3, 42.0, 41.4, 40.5, 40.4, 37.7, 34.9, 29.3, 23.9, 23.6, 21.5, 21.4, 21.2, 19.7, 18.3, 16.3, 15.1, 10.5, 9.3.

### EXAMPLE 3E

A solution of EXAMPLE 3C (815 mg) in dichloromethane (20 mL) and trifluoroacetic acid (1 mL) at 25°C was stirred for one hour, treated with more trifluoroacetic acid (1 mL), stirred for another hour, washed with saturated aqueous NaHCO₃, and dried (Na₂SO₄), filtered, and concentrated; and the concentrate was flash chromatographed on silica gel with 10-40% acetone/hexane. ¹H NMR (CDCl₃) δ 8.05 (m, 2H), 7.55 (m, 1H), 7.43 (m, 2H), 5.05 (dd, J=10.5, 7.8Hz, 1H), 4.75 (d, J=7.8Hz, 1H), 4.59 (dd, J=10.5, 2.1Hz, 1H), 4.00 (m, 2H), 3.71 (d, J=2.7Hz, 1H), 3.61-3.45 (m, 4H), 2.91 (m, 3H), 2.70 (m, 2H), 2.66 (s, 3H), 2.40 (t, J=2.4Hz, 1H), 2.32 (m, 1H), 2.28 (s, 6H), 1.77 (m, 2H), 1.67 (s, 3H), l. 60 (m, 2H), 1.54 (s, 3H), 1.51 (m, 2H), 1.44 (d, J=3.3Hz, 3H), 1.28 (d, J=6.0Hz, 3H), 1.16 (d, 3H, J=6.9Hz), 0.92 (d, J=6.9Hz, 3H), 0.81 (d, J=7.2Hz 3H), 0.79 (t, J=7.5Hz, 3H).

### EXAMPLE 3F

A solution of EXAMPLE 3E (612 mg) and triacetoxy periodinane (430 mg) in dichloromethane (10 mL) at 25°C was stirred for 1 hour, diluted with dichloromethane, washed sequentially with saturated aqueous NaHCO₃, saturated aqueous sodium thiosulfate, and brine, and dried (Na₂SO₄), filtered, and concentrated. ¹H NMR (CDCl₃) δ 8.02 (m, 2H), 7.56 (m, 1H), 7.44 (m, 2H), 5.03 (dd, J=10.5, 7.5Hz, 1H), 4.57 (dd, J=9.9, 2.4Hz, 1H), 4.51 (d, J=7.5,Hz, 1H), 4.20 (d, J=7.5Hz, 1H), 3.76 (q, J=7.2Hz, 1H), 3.70 (t, J=2.1Hz, 2H), 3.62 (m, 1H), 3.53 (d, J=8.1Hz, 1H), 3.45 (d, J=10.5Hz, 1H), 3.04-2.80 (m, 4H), 2.68 (d, J=10.5Hz, 1H), 2.64 (s, 3H), 2.36 (t, J=2.1Hz, 1H), 2.27 (s, 6H), 2.17 (dd, J=15, 9Hz, 1H), 1.80 (m, 2H), 1.70 (s, 3H), 1.60 (m, 3H), 1.49 (s, 3H), 1.47 (s, 3H), 1.33 (d, 3H, J=7.2Hz), 1.30 (d, J=5.7Hz, 3H), 1.00 (d, J=3.3Hz, 3H), 0.98 (d, J=3.3Hz, 3H), 0.80 (t, J=7.2Hz, 3H).

### EXAMPLE 3G

### (2aR, 4aS, 6R, 8S, 9R, 10R, 12R, 15R, 15aS, 15bS)-15-ethyl-3,4a,6,8,10,12,15a-heptamethyl-2,5,11,13-tetraoxo-8-(prop-2-ynyloxy)hexadecahydro-2H-1,14-dioxa-3-azacyclotetradeca-(1,2,3-cd)pentalen-9-yl 3,4,6-trideoxy-3-(dimethylamino)-β-D-xylo-hexopyranoside

A solution of EXAMPLE 3F (60 mg) in methanol (5 mL) was heated at 60°C for 36 hours and concentrated; and the concentrate was flash chromatographed on silica gel with 84:15:1 of dichloromethane/methanol/concentrated ammonium hydroxide. ¹³C NMR (CDCl₃) δ 216.9, 205.8, 176.5, 170.4, 104.0, 85.7, 83.9, 80.4, 80.2, 78.8, 77.4, 74.4, 70.4, 69.5, 67.5, 66.1, 65.8, 56.6, 52.7, 51.4, 50.7, 48.5, 41.9, 40.3, 39.2, 36.6, 28.5, 22.3, 21.8, 21.2, 19.6, 16.9, 15.8, 14.3, 10.4.

### EXAMPLE 4A

A solution of EXAMPLE 3F (520 mg), triethylamine (5 mL), 2-(5-bromothien-2-yl)pyridine (250 mg), tris(dibenzylideneacetone)dipalladium(0) (55 mg), bis(1,2-diphenylphosphino)ethane (48 mg), and copper(I) iodide (4 mg) in acetonitrile (10 mL) at 80°C was stirred for 3 hours, cooled to room temperature, and concentrated; and the concentrate was flash chromatographed on silica gel with 10-40% acetone/hexane. ¹H NMR (CDCl₃) δ 8.56 (m, 1H), 8.03 (m, 2H), 7.73-7.54 (m, 3H), 7.47-7.41 (m, 3H), 7.17 (m, 1H), 7.13 (d, J=4.2Hz, 1H), 5.05 (dd, J=10.5, 7.2Hz, 1H), 4.59 (dd, J=10.5, 2.7Hz, 1H), 4.53 (d, J=7.8Hz, 1H), 4.29 (d, J=8.1Hz, 1H), 3.95 (s, 2H), 3.80 (q, J=6.9Hz, 1H), 3.62 (m, 1H), 3.48 (d, J=8.1Hz, 1H), 3.41 (d, J=10.2Hz, 1H), 3.06 (quintet, J=7.5Hz, 1H), 2.91 (d, J=8.1Hz, 1H), 2.95-2.80 (m, 2H), 2.67 (d, J=10.5Hz, 1H), 2.52 (s, 3H), 2.28 (s, 6H), 2.23 (m, 1H), 1.80 (m, 2H), 1.72 (s, 3H), 1.60 (m, 1H), 1.54 (s, 3H), 1.50 (m, 2H), 1.48 (s, 3H), 1.35 (d, J=6.9Hz, 3H), 1.31 (d, J=5.7Hz, 3H), 1.02 (d, J=2.7Hz, 3H), 1.00 (d, J=3.3Hz, 3H), 0.81 (t, J=7.5Hz, 3H).

### EXAMPLE 4B

### (2aR,4aS,6R,8S,9R,10R,12R,15R,15aS,15bS)-15-ethyl-3,4a,6,8,10,12,15a-heptamethyl-2,5,11,13-tetraoxo-8-((3-(5-(pyridin-2-yl)thien-2-yl)prop-2-ynyl)oxy)hexadecahydro-2H-1,14-dioxa-3-azacyclotetradeca(1,2,3-cd)pentalen-9-yl 3,4,6-trideoxy-3-(dimethylamino)-β-D-xylo-hexopyranoside

A solution of EXAMPLE 4A (445 mg) in methanol (20 mL) was refluxed for 20 hours and concentrated; and the concentrate was flash chromatographed on silica gel with 90:10:0.5 dichloromethane/methanol/concentrated ammonium hydroxide. ¹³C NMR (CDCl₃) δ 217.1, 205.9, 176.6, 170.6, 151.7, 149.7, 146.0, 136.7, 132.6, 124.5, 124.1, 122.4, 118.7, 104.1, 94.4, 85.8, 80.3, 80.2, 79.7, 78.5, 70.4, 69.6, 67.4, 66.1, 65.8, 56.6, 52.7, 52.4, 50.6, 48.6, 42.2, 40.3, 39.6, 37.0, 28.4, 22.7, 22.5, 21.7, 21.3, 19.7, 16.9, 15.9, 14.3, 10.4.

### EXAMPLE 5

### (2aR,4aS,6R,8S,9R,10R,12R,15R,15aS,15bS)-3-allyl-15-ethyl-8-methoxy-4a,6,8,10,12,15a-hexamethyl-2,5,13-trioxo-9-((3,4,6-trideoxy-3-(dimethylamino)-β-D-xylohexopyranosyl)oxy)hexadecahydro-2H-1,14-dioxa-3-azacyclotetradeca(1,2,3-cd)pentalen-11-yl 2,6-dideoxy-3-C-methyl-3-O-methyl-α-L-ribo-hexopyranoside; This example was prepared as described in SCHEME 3.

### EXAMPLE 6A

A solution of EXAMPLE 5 (1.13 g), triethylamine (0.5 mL), and 4-(N,N-dimethylamino)pyridine (10 mg) in dichloromethane (80 mL) at 0°C was treated with chloroacetic anhydride (1.18 g), stirred at 25°C for 3 hours, washed with saturated aqueous NaHCO₃, and dried (Na₂SO₄), filtered, and concentrated; and the concentrate was flash chromatographed on silica gel with 30% acetone/hexane.

### EXAMPLE 6B

A solution of EXAMPLE 6A (4.08g) and allylamine (2.5 mL) at 25°C was stirred for 20 hours and concentrated; and the concentrate was flash chromatographed on silica gel with 89:10:1 dichloromethane/methanol/concentrated ammonium hydroxide.

### EXAMPLE 6C

A solution of EXAMPLE 6B (2.77 g), 37% aqueous formaldehyde (160 µL) and acetic acid (10 drops) in toluene (80 mL) was stirred for 30 minutes at 25°C and at 110°C for 1.5 hours under a Dean-Stark trap, and concentrated; and the concentrate was flash chromatographed on silica gel with 10-50% acetone/hexane.

A solution of the product from the preceeding paragraph (2.48 g) in THF (50 mL) at 25°C was treated with 1M tetrabutylammonium fluoride in THF (3.3 mL), stirred for 2 hours, and concentrated. The concentrate was dissolved in dichloromethane (60 mL), treated with triacetoxy periodinane (3.56 g), stirred at 25°C for 2 hours, diluted with dichloromethane, washed sequentially with saturated aqueous NaHCO₃, saturated aqueous sodium thiosulfate, and brine, and dried (Na₂SO₄), filtered and concentrated; and the concentrate was flash chromatographed on silica gel with 1:1 of acetone/hexane.

### EXAMPLE 6D

A solution of EXAMPLE 6C (100mg), tetrakis palladium(0) (triphenylphosphine) (16 mg), and N,N-dimethylbarbituric acid (65 mg) in dichloromethane (1 mL) at 35°C was stirred for 5 hours, treated with dichloromethane (70 mL), washed with saturated aqueous NaHCO₃ and brine, and dried (Na₂SO₄), filtered and concentrated; and the concentrate was flash chromatographed on silica gel with 29.5:70:0.5% acetone/hexane/ triethylamine.

### EXAMPLE 6E

### (2aR, 4aS, 6R, 8S, 9R, 10R, 12R, 15R, 15aS, 15bS) -15-ethyl-8-methoxy-4a,6,8,10,12,15a-hexamethyl-2,5,11,13-tetraoxohexadecahydro-2H-1,14-dioxa-3-azacyclotetradeca(1,2,3-cd)-pentalen-9-yl 3,4,6-trideoxy-3-(dimethylamino)-β-D-xylohexopyranoside

A solution of EXAMPLE 6D in methanol (1 mL) at 25°C was stirred for 24 hours and concentrated; and the concentrate was flash chromatographed on silica gel with 94.5:5:0.5 dichloromethane/methanol/concentrated ammonium hydroxide. ¹³C NMR (CDCl₃) δ 217.2, 205.5, 177.0, 170.3, 104.0, 86.4, 79.3, 78.5, 78.1, 70.4, 69.5, 65.7, 60.3, 59.2, 56.6, 52.6, 51.1, 50.6, 48.6, 40.8, 40.2, 36.7, 28.4, 21.6, 21.4, 21.2, 21.0, 19.8, 16.9, 16.1, 14.4, 10.3.

### EXAMPLE 7A

A solution of EXAMPLE 6A (480 mg) and 2M methylamine in THF (1.5 mL) in N,N-dimethylformamide (3 mL) was stirred at 25°C for 6 hours and concentrated.

### EXAMPLE 7B

A solution of EXAMPLE 7A (76 mg), 37% aqueous formaldehyde (7.2 µL), and acetic acid (one drop) in toluene (20 mL) was stirred for 30 minutes at 25°C and at 110°C for 1.5 hours under a Dean-Stark trap, and concentrated; and the concentrate was flash chromatographed on silica gel with 10-50% acetone/hexane.

### EXAMPLE 7C

A solution of EXAMPLE 7B (283 mg) in THF (5 mL) at 25°C was treated with 1M tetrabutylammonium fluoride in THF (0.4 mL), stirred for 1.5 hours, and concentrated. A solution of the concentrate (260 mg) in dichloromethane (8 mL) at 25°C was treated with triacetoxy periodinane (190 mg), stirred for 1 hour, diluted with dichloromethane, washed with saturated aqueous NaHCO₃, saturated aqueous sodium thiosulfate, and brine, and dried (Na₂SO₄), filtered and concentrated; and the concentrate was flash chromatographed on silica gel with 1:1 acetone/hexane.

### EXAMPLE 7D

### (2aR,4aS,6R,8S,9R,10R,12R,15R,15aS,15bS)-15-ethyl-8-methoxy-3,4a,6,8,10,12,15a-heptamethyl-2,5,11,13-tetraoxohexadecahydro-2H-1,14-dioxa-3-azacyclotetradeca-(1,2,3-cd)pentalen-9-yl 3,4,6-trideoxy-3-(dimethylamino)-β-D-xylo-hexopyranoside

A solution of EXAMPLE 7C (225 mg) in methanol (5 mL) at 55°C was stirred for 3 hours and concentrated; and the concentrate was flash chromatographed on silica gel with 89/10/1 dichloromethane/methanol/concentrated ammonium hydroxide.

## Claims

1. A compound having formula (I) 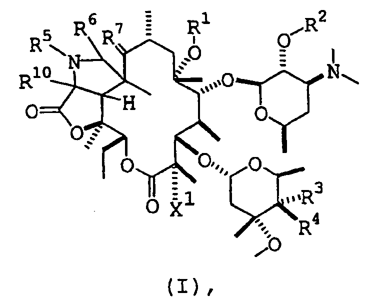 or formula (II), 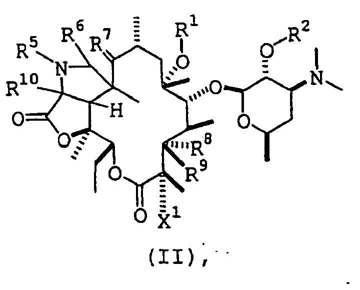 or a pharmaceutically acceptable salt thereof, in which
R¹ is hydrogen, -R¹¹, -C (O) OR¹¹, -C (O) NH², -C (O) NHR¹², or -C(O)NR¹² R¹³;
R² is hydrogen or R^{P}, in which R^{P} is a hydroxyl protecting moiety;
one of R³ or R⁴ is hydrogen and the other is -OH, -OR^{P}, or -OC (O) R¹⁵; or
R³ and R⁴ together are =O or -CH₂O-;
R⁵ is hydrogen, -R¹⁹, -C (O) OR¹⁹, -C(O)NH₂, -C (O) NHR²⁰, or -C (O) NR²⁰R²¹:
R⁶ and R¹⁰ are independently hydrogen or -R²³;
R⁷ is =O, =NOH, =NOR^{P}, or =NOR²⁴
one of R⁸ and R⁹ is hydrogen, and the other is -OH or -OR³²; or
R⁸ and R⁹ together are =O;
R¹¹, R¹⁵, R¹⁹, R²⁴, and R²⁶ are independently alkyl, -(CH₂)alkenyl, -(CH₂)alkynyl, alkyl substituted with one or two or three substituents independently selected from the group consisting of cycloalkyl, halo, aryl, heteroaryl, and heterocyclyl, -(CH₂)alkenyl substituted with one or two or three substituents independently selected from the group consisting of cycloalkyl, halo, aryl, heteroaryl, and heterocyclyl, or -(CH₂)alkynyl substituted with one or two or three substituents independently selected from the group consisting of cycloalkyl, aryl, heteroaryl, and heterocyclyl;
R¹², R¹³, R²⁰, R²¹, R²⁷, and R²⁸ are independently alkyl, cycloalkyl, -(CH₂) alkenyl, -(CH₂) alkynyl, cycloalkyl, aryl, heteroaryl, heterocyclyl, alkyl substituted with one substituent selected from the group consisting of cycloalkyl, aryl, heteroaryl, heterocyclyl, -NH₂, -NHR³⁰, and -NR³⁰R³¹, -(CH₂)alkenyl substituted with one substituent selected from the group consisting of cycloalkyl, aryl, heteroaryl, heterocyclyl, -NH₂, -NHR³⁰, and -NR³⁰R³¹, or -(CH₂)alkynyl substituted with one substituent selected from the group consisting of cycloalkyl, aryl, heteroaryl, heterocyclyl, -NH₂, -NHR³⁰, and -NR³⁰R³¹;
R²³ is alkyl, alkenyl, alkynyl, aryl, heteroaryl, heterocyclyl, alkyl substituted with one or two or three substituents independently selected from the group consisting of cycloalkyl, halo, aryl, heteroaryl, and heterocyclyl, alkenyl substituted with one or two or three substituents independently selected from the group consisting of cycloalkyl, halo, aryl, heteroaryl, and heterocyclyl, or alkynyl substituted with one or two or three substituents independently selected from the group consisting of cycloalkyl, aryl, heteroaryl, and heterocyclyl;
R³⁰ and R³¹ are independently alkyl, cycloalkyl, aryl, heteroaryl, heterocyclyl, -(CH₂)alkenyl, -(CH₂)alkynyl, cycloalkyl, alkyl substituted with one substituent selected from the group consisting of cycloalkyl, aryl, heteroaryl, heterocyclyl, -NH₂, -NH(alkyl), and -N(alkyl)₂, -(CH₂)alkenyl substituted with one substituent selected from the group consisting of cycloalkyl, aryl, heteroaryl, heterocyclyl, -NH₂, -NH(alkyl), and -N(alkyl)₂, or -(CH₂)alkynyl substituted with one substituent selected from the group consisting of cycloalkyl, aryl, heteroaryl, heterocyclyl, -NH₂, -NH(alkyl), and -N(alkyl)₂;
R³² is -R²⁶, -C(O)OR²⁶, -C (O) NH₂, -C (O) NHR²⁷, or -C(O)NR²⁷R²⁸; and
X¹ is hydrogen or fluoride.

2. The compound of claim 1 having formula (I) or formula (II), or a pharmaceutically acceptable salt thereof, in which R¹ is methyl, prop-2-ynyl, 3-(5-pyridin-2-ylthien-2-yl)prop-2-ynyl, 3-(quinolin-3-yl)prop-2-enyl, 3-(3-pyridin-2-ylisoxazol-5-yl)prop-2-ynyl, or 3-(5-pyrimidin-2-ylthien-2-yl)prop-2-ynyl; R² is hydrogen; R³ is -OH, ((phenyl)carbonyl)oxy, ((methyl)carbonyl)oxy, (trimethylsilyl)oxy, or (triethylsilyl)oxy and R⁴ is hydrogen, or R³ and R⁴ together are =O or -CH₂O-; R⁵ is hydrogen, methyl, ethyl, propyl, prop-2-ynyl, prop-2-enyl, phenylmethyl, 4-methoxyphenylmethyl, or 2,4-dimethoxyphenylmethyl; R⁶ is hydrogen, methyl, ethyl, ethenyl, or phenyl; R⁷ is =O; R⁸ is hydrogen and R⁹ is -OH, or R⁸ and R⁹ together are =O; R¹⁰ is hydrogen, methyl, ethyl, prop-2-ynyl or prop-2-enyl; and X¹ is hydrogen or fluoride.

3. The compound of claim 1 having formula (I) or formula (II), or a pharmaceutically acceptable salt thereof, in which R¹ is alkyl, -(CH₂)alkynyl, or -(CH₂)alkynyl substituted with thienyl, in which the thienyl is substituted with pyridyl; R² is hydrogen; R³ is ((phenyl)carbonyl)oxy; R⁴ is hydrogen; R⁵ is hydrogen or alkyl; R⁶ is hydrogen; R⁷ is =O; R⁸ and R⁹ together are =O; R¹⁰ is hydrogen; and X¹ is hydrogen.

4. The compound of claim 1 having formula (I) or formula (II), or a pharmaceutically acceptable salt thereof, in which R¹ is methyl, prop-2-ynyl or prop-2-ynyl substituted with thienyl, in which the thienyl is substituted with pyridyl; R² is hydrogen; R³ is ((phenyl)carbonyl)oxy; R⁴ is hydrogen; R⁵ is hydrogen or methyl; R⁶ is hydrogen; R⁷ is =O; R⁸ and R⁹ together are =0; R¹⁰ is hydrogen; and X¹ is hydrogen.

5. The compound of claim 3 having formula (I) or formula (II), or a pharmaceutically acceptable salt thereof, in which R¹ is methyl; R² is hydrogen; R³ is ((phenyl)carbonyl)oxy; R⁴ is hydrogen; R⁵ is hydrogen or methyl; R⁶ is hydrogen; R⁷ is =O; R⁸ and R⁹ together are =O; R¹⁰ is hydrogen; and X¹ is hydrogen.

6. The compound of claim 3 having formula (I) or formula (II), or a pharmaceutically acceptable salt thereof, in which R¹ is prop-2-ynyl; R² is hydrogen; R³ is ((phenyl)carbonyl)oxy; R⁴ is hydrogen; R⁵ is hydrogen or methyl; R⁶ is hydrogen; R⁷ is =O; R⁸ and R⁹ together are =0; R¹⁰ is hydrogen; and X¹ is hydrogen.

7. The compound of claim 3 having formula (I) or formula (II), or a pharmaceutically acceptable salt thereof, in which R¹ is 3-(5-pyridin-2-ylthien-2-yl)prop-2-ynyl; R² is hydrogen; R³ is ((phenyl)carbonyl)oxy; R⁴ is hydrogen; R⁵ is hydrogen or methyl; R⁶ is hydrogen; R⁷ is =O; R⁸ and R⁹ together are =O; R¹⁰ is hydrogen; and X¹ is hydrogen.

8. A composition for prophylaxis or treatment of bacterial infections in a fish or a mammal comprising a therapeutically effective amount of a compound of claim 1 and an excipient.

9. Use of a compound of claim 1 for preparation of a medicament for prophylaxis or treatment of bacterial infections.

10. The compound of claim 1, or a pharmaceutically acceptable salt thereof, which is
(2aR,4aS,6R,8S,9R,10R,12R,15R,15aS,15bS)-15-ethyl-3,4a,6,8,10,12,15a-heptamethyl-2,5,11,13-tetraoxo-8-((3-(5-(pyridin-2-yl)thien-2-yl)prop-2-ynyl)oxy)hexadecahydro-2H-1,14-dioxa-3-azacyclotetradeca(1,2,3-cd)pentalen-9-yl 3,4,6-trideoxy-3-(dimethylamino)-β-D-xylo-hexopyranoside;
(2aR,4aS,6R,8S,9R,10R,12R,15R,15aS,15bS)-15-ethyl-8-methoxy-3,4a,6,8,10,12,15a-heptamethyl-2,5,11;13-tetraoxohexadecahydro-2H-1,14-dioxa-3-azacyclotetradeca-(1,2,3-cd)pentalen-9-yl 3,4,6-trideoxy-3-(dimethylamino)-β-D-xylo-hexapyranoside;
(2aS,4aR,6R,8S,9R,10R,12R,15R,15aS,15bR)-15-ethyl-8-methoxy-3,4a,6,8,10,12,15a-heptamethyl-2,5,11,13-tetraoxohexadecahydro-2H-1,14-dioxa-3-azacyclotetradeca-(1,2,3-cd)pentalen-9-yl 3,4,6-trideoxy-3-(dimethylamino)-β-D-xylo-hexopyranoside;
(2aR,4aS,6R,8S,9R,10S,11S,12R,15R,15aS,15bS)-15-ethyl-3,4a,6,8,10,12,15a-heptamethyl-2,5,13-trioxo-8-(prop-2-ynyloxy)-9-((3,4,6-trideoxy-3-(dimethylamino)-β-D-xylohexopyranosyl)oxy)hexadecahydro-2H-1,14-dioxa-3-azacyclotetradeca(1,2,3-cd)pentalen-11-yl 4-O-benzoyl-2, 6-dideoxy-3-C-methyl-3-O-methyl-α-L-ribo-hexopyranoside;
(2aR,4aS,6R,8S,9R,10R,12R,15R,15aS,15bS)-15-ethyl-3,4a,6,8,10,12,15a-heptamethyl-2,5,11,13-tetraoxo-8-(prop-2-ynyloxy)-hexadecahydro-2H-1,14-dioxa-3-azacyclotetradeca-(1,2,3-cd)pentalen-9-yl 3,4,6-trideoxy-3-(dimethylamino)-β-D-xylo-hexopyranoside; or
(2aR,4aS,6R,8S,9R,10R,12R,15R,15aS,15bS)-15-ethyl-8-methoxy-4a,6,8,10,12,15a-hexamethyl-2,5,11,13-tetraoxo hexadecahydro-2H-1,14-dioxa-3-azacyclotetradeca (1,2,3-cd)pentalen-9-yl 3,4,6-trideoxy-3-(dimethylamino)-β-D-xylo-hexopyranoside.

## Patentansprüche

1. Eine Verbindung mit der Formel (I) 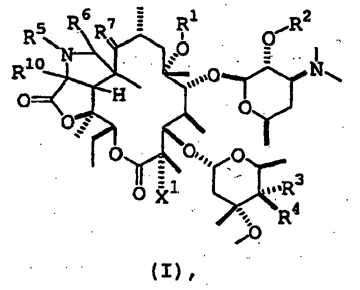 oder Formel (II) 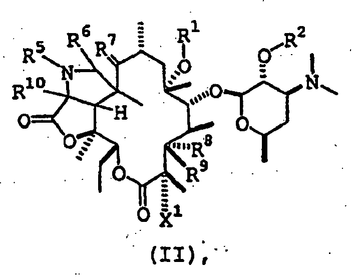 oder ein pharmazeutisch verträgliches Salz davon, worin
R¹ Wasserstoff, -R¹¹, -C(O)OR¹¹, -C(O)NH₂, -C(O)NHR¹² oder -C (O) NR¹²R¹³ ist;
R² ist Wasserstoff oder R^{P}, worin R^{P} ein Hydroxylschützender Teil ist;
eines von R³ oder R⁴ ist Wasserstoff und das andere ist -OH -OR^{p} oder -OC(O)R¹⁵; oder
R³ und R⁴ zusammen sind =O oder -CH₂O-;
R⁵ ist Wasserstoff, -R¹⁹, -C(O)OR¹⁹, -C(O)NH₂, -C(O)NHR²⁰ oder -C(O)NR²⁰R²¹ ;
R⁶ und R¹⁰ sind unabhängig Wasserstoff oder -R²³;
R⁷ ist =O, =NOH, =NOR^{P} oder =NOR²⁴;
eines von R⁸ und R⁹ ist Wasserstoff und das andere ist -OH oder -OR³²; oder
R⁸ und R⁹ zusammen sind =0;
R¹¹, R¹⁵, R¹⁹, R²⁴ und R²⁶ sind unabhängig Alkyl, - (CH₂)Alkenyl, -(CH₂)Alkinyl, Alkyl substituiert mit einem oder zwei oder drei Substituenten unabhängig gewählt aus der Gruppe bestehend aus Cycloalkyl, Halo, Aryl, Heteroaryl und Heterocyclyl, -(CH₂)Alkenyl substituiert mit einem oder zwei oder drei Substituenten unabhängig gewählt aus der Gruppe bestehend aus Cycloalkyl, Halo, Aryl, Heteroaryl und Heterocyclyl oder - (CH₂)Alkinyl substituiert mit einem oder zwei oder drei Substituenten unabhängig gewählt aus der Gruppe bestehend aus Cycloalkyl, Aryl, Heteroaryl und Heterocyclyl;
R¹², R¹³, R²⁰, R²¹, R²⁷ und R²⁸ sind unabhängig Alkyl, Cycloalkyl, -(CH₂)Alkenyl, -(CH₂)Alkinyl, Cycloalkyl, Aryl, Heteroaryl, Heterocyclyl, Alkyl substituiert mit einem Substituenten gewählt aus der Gruppe bestehend aus Cycloalkyl, Aryl, Heteroaryl, Heterocyclyl, -NH₂, -NHR³⁰ und -NR³⁰R³¹, - (CH₂)Alkenyl substituiert mit einem Substituenten gewählt aus der Gruppe bestehend aus Cycloalkyl, Aryl, Heteroaryl, Heterocyclyl, -NH₂, -NHR³⁰ und -NR³⁰R³¹, oder -(CH₂)Alkinyl substituiert mit einem Substituenten gewählt aus der Gruppe bestehend aus Cycloalkyl, Aryl, Heteroaryl, Heterocyclyl, -NH₂, -NHR³⁰ und -NR³⁰R³¹;
R²³ ist Alkyl, Alkenyl, Alkinyl, Aryl, Heteroaryl, Heterocyclyl, Alkyl substituiert mit einem oder zwei oder drei Substituenten unabhängig gewählt aus der Gruppe bestehend aus Cycloalkyl, Halo, Aryl, Heteroaryl und Heterocyclyl, Alkenyl substituiert mit einem oder zwei oder drei Substituenten unabhängig gewählt aus der Gruppe bestehend aus Cycloalkyl., Halo, Aryl, Heteroaryl und Heterocyclyl, oder Alkinyl substituiert mit einem oder zwei oder drei Substituenten unabhängig gewählt aus der Gruppe bestehend aus Cycloalkyl, Aryl, Heteroaryl und Heterocyclyl;
R³⁰ und R³¹ sind unabhängig Alkyl, Cycloalkyl, Aryl, Heteroaryl, Heterocyclyl, -(CH₂)Alkenyl, -(CH₂)Alkinyl, Cycloalkyl, Alkyl substituiert mit einem Substituenten gewählt aus der Gruppe bestehend aus Cycloalkyl, Aryl, Heteroaryl, Heterocyclyl, -NH₂, -NH (Alkyl), und -N(Alkyl)₂, - (CH₂) Alkenyl substituiert mit einem Substituenten gewählt aus der Gruppe bestehend aus Cycloalkyl, Aryl, Heteroaryl, Heterocyclyl, -NH₂, -NH(Alkyl) und -N(Alkyl)₂ oder -(CH₂)Alkinyl substituiert mit einem Substituenten gewählt aus der Gruppe bestehend aus Cycloalkyl, Aryl, Heteroaryl, Heterocyclyl, -NH₂, -NH(Alkyl) und -N (Alkyl) ₂;
R³² ist -R²⁶, -C(O)OR²⁶, -C(O)NH₂, -C(O)NHR²⁷ oder -C(O)NR²⁷R²⁸; und
X¹ ist Wasserstoff oder Fluorid.

2. Die Verbindung von Anspruch 1 mit der Formel (I) oder Formel (II) oder ein pharmazeutisch verträgliches Salz davon, worin R¹ Methyl ist, Prop-2-ynyl, 3-(5-Pyridin-2-ylthien-2-yl)prop-2-ynyl, 3-(Chinolin-3-yl)prop-2-enyl, 3-(3-Pyridin-2-ylisoxazol-5-yl)prop-2-ynyl, oder 3-(5-Pyrimidin-2-ylthien-2-yl)prop-2-ynyl; R² ist Wasserstoff; R³ ist -OH, ((Phenyl)carbonyl)oxy, ((Methyl)carbonyl)oxy, (Trimethylsilyl)oxy oder (Triethylsilyl)oxy, und R⁴ ist Wasserstoff, oder R³ und R⁴ zusammen sind =O oder -CH₂O-; R⁵ ist Wasserstoff, Methyl, Ethyl, Propyl, Prop-2-ynyl, Prop-2-enyl, Phenylmethyl, 4-Methoxyphenylmethyl oder 2,4-Dimethoxyphenylmethyl; R⁶ ist Wasserstoff, Methyl, Ethyl, Ethenyl oder Phenyl; R⁷ ist =O; R⁸ ist Wasserstoff und R⁹ ist -OH, oder R⁸ und R⁹ zusammen sind =0; R¹⁰ ist Wasserstoff, Methyl, Ethyl, Prop-2-ynyl oder Prop-2-enyl; und X¹ ist Wasserstoff oder Fluorid.

3. Die Verbindung von Anspruch 1 mit der Formel (I) oder Formel (II) oder ein pharmazeutisch verträgliches Salz davon, worin R¹ Alkyl ist, -(CH₂)Alkinyl oder -(CH₂)Alkinyl substituiert mit Thienyl, worin das Thienyl mit Pyridyl substituiert ist; R² ist Wasserstoff; R³ ist ((Phenyl)carbonyl)oxy; R⁴ ist Wasserstoff; R⁵ ist Wasserstoff oder Alkyl; R⁶ ist Wasserstoff; R⁷ ist =O; R⁸ und R⁹ sind zusammen =O; R¹⁰ ist Wasserstoff; und X¹ ist Wasserstoff.

4. Die Verbindung von Anspruch 1 mit der Formel (I) oder Formel (II) oder ein pharmazeutisch verträgliches Salz davon, worin R¹ Methyl ist, Prop-2-ynyl oder Prop-2-ynyl substituiert mit Thienyl, worin das Thienyl mit Pyridyl substituiert ist; R² ist Wasserstoff; R³ ist ((Phenyl)carbonyl)oxy; R⁴ ist Wasserstoff; R⁵ ist Wasserstoff oder Methyl; R⁶ ist Wasserstoff; R⁷ ist =O, R⁸ und R⁹ sind zusammen =0; R¹⁰ ist Wasserstoff; und X¹ ist Wasserstoff.

5. Die Verbindung von Anspruch 3 mit der Formel (I) oder Formel (II) oder ein pharmazeutisch verträgliches Salz davon, worin R¹ Methyl ist; R² ist Wasserstoff; R³ ist ((Phenyl)carbonyl)oxy; R⁴ ist Wasserstoff; R⁵ ist Wasserstoff oder Methyl; R⁶ ist Wasserstoff; R⁷ ist =O; R⁸ und R⁹ sind zusammen. =O; R¹⁰ ist Wasserstoff; und X¹ ist Wasserstoff.

6. Die Verbindung von Anspruch 3 mit der Formel (I) oder Formel (II) oder ein pharmazeutisch verträgliches Salz davon,
worin R¹ Prop-2-ynyl ist; R² ist Wasserstoff; R³ ist ((Phenyl)carbonyl)oxy; R⁴ ist Wasserstoff; R⁵ ist Wasserstoff oder Methyl; R⁶ ist Wasserstoff; R⁷ ist =O; R⁸ und R⁹ sind zusammen =O; R¹⁰ ist Wasserstoff; und X¹ ist Wasserstoff.

7. Die Verbindung von Anspruch 3 mit der Formel (I) oder Formel (II) oder ein pharmazeutisch verträgliches Salz davon, worin R¹ 3-(5-Pyridin-2-ylthien-2-yl)prop-2-ynyl) ist; R² ist Wasserstoff; R³ ist ((Phenyl)carbonyl)oxy; R⁴ ist Wasserstoff; R⁵ ist Wasserstoff oder Methyl; R⁶ ist Wasserstoff; R⁷ ist =O; R⁸ und R⁹ sind zusammen =O; R¹⁰ ist Wasserstoff; und X¹ ist Wasserstoff.

8. Eine. Zusammensetzung zur Prophylaxe und Behandlung von bakteriellen Infektionen in einem Fisch oder einem Säugetier, die eine therapeutisch wirksame Menge einer Verbindung von Anspruch 1 und ein Bindemittel umfasst.

9. Verwendung einer Verbindung von Anspruch 1 zur Herstellung eines Medikaments zur Prophylaxe oder Behandlung von bakteriellen Infektionen.

10. Die Verbindung von Anspruch 1 oder ein pharmazeutisch verträgliches Salz davon, die folgendes ist:
(2aR, 4aS, 6R, 8S, 9R, 10R, 12R, 15R, 15aS, 15bS) -15-Ethyl-3,4a,6,8,10,12,15a-heptamethyl-2,5,11,13-tetraoxo-8-((3-(5-(pyridin-2-yl)thien-2-yl)prop-2-ynyl)oxy)hexadecahydro-2H-1,14-dioxa-3-azacyclotetradeca(1,2,3-cd)pentalen-9-yl 3,4,6-Trideoxy-3-(dimethylamino)-β-D-xylo-hexopyrosid;
(2aR,4aS,6R,8S,9R,10R,12R,15R,15aS,15bS)-15-Ethyl-8-methoxy-3,4a,6,8,10,12,15a-heptamethyl-2,5,11,13-tetraoxohexadecahydro-2H-1,14-dioxa-3-azacyclotetradeca(1,2,3-cd)pentalen-9-yl 3,4,6-Trideoxy-3-(dimethylamino)-β-D-xylohexopyranosid;
(2aS, 4aR, 6R, 8S, 9R, 10R, 12R, 15R, 15aS, 15bR) -15-Ethyl-8-methoxy-3,4a,6,8,10,12,15a-heptamethyl-2,5,11,13-tetraoxohexadecahydro-2H-1,14-dioxa-3-azacyclotetradeca(1, 2, 3-cd)pentalen-9-yl 3,4,6-Trideoxy-3-(dimethylamino)-β-D-xylohexopyranosid;
(2aR,4aS,6R,8S,9R,10S,11S,12R,15R,15aS,15bS)-15-Ethyl-3,4a,6,8,10,12,15a-heptamethyl-2,5,13-trioxo-8-(prop-2-ynyloxy)-9-((3,4,6-trideoxy-3-(dimethylamino)-β-D-xylohexopyranosyl)oxy)hexadecahydro-2H-1,14-dioxa-3-azacyclotetradeca(1,2,3-cd)pentalen-11-yl 4-O-Benzoyl-2, 6-dideoxy-3-C-methyl-3-O-methyl-α-L-ribo-hexopyranosid;
(2aR,4aS,6R,8S,9R,10R,12R,15R,15aS,15bS)-15-Ethyl-3, 4a, 6, 8, 10, 12, 15a-heptamethyl-2, 5, 11, 13-tetraoxo-8-(prop-2-ynyloxy)-hexadecahydro-2H-1,14-dioxa-3-azacyclotetradeca(1,2,3-cd)pentalen-9-yl 3, 4, 6-Trideoxy-3-(dimethylamino)-β-D-xylohexopyranosid; oder
(2aR, 4aS, 6R, 8S, 9R, 10R, 12R, 15R, 15aS, 15bS) -15-Ethyl-8-methoxy-4a,6,8,10,12,15a-hexamethyl-2,5,11,13-tetraoxohexadecahydro-2H-1,14-dioxa-3-azacyclotetradeca(1,2,3-cd)pentalen-9-yl 3,4,6-Trideoxy-3-(dimethylamino)-β-D-xylohexopyranosid.

## Revendications

1. Composé présentant la formule (I) 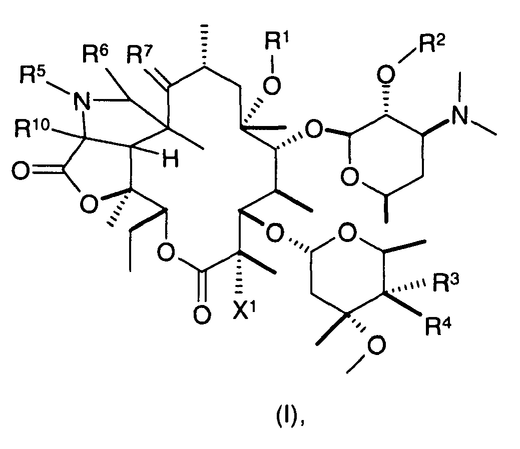 ou la formule (II) 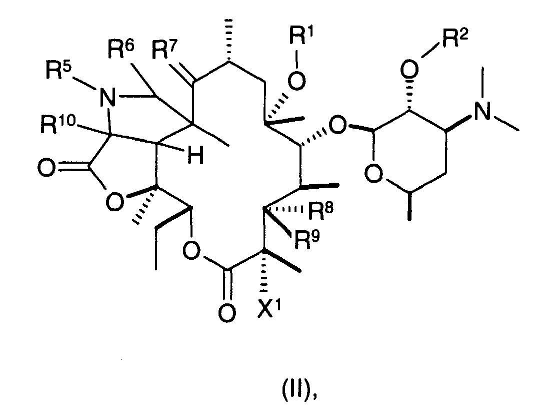 ou sel pharmaceutiquement acceptable de celui-ci,
dans lesquelles
R¹ est un atome d'hydrogène, -R¹¹, -C(O)OR¹¹, -C(O)NH₂, -C(O)NHR¹² ou -C(O)NR¹²R¹³ ;
R² est un atome d'hydrogène ou R^{P}, où R^{P} est une moitié hydroxyle-protectrice ;
un parmi R³ ou R⁴ est un atome d'hydrogène et l'autre est -OH, -OR^{P}, ou -OC(O)R¹⁵ ; ou
R³ et R⁴ sont ensemble =O ou -CH₂O- ;
R⁵ est un atome d'hydrogène, -R¹⁹, -C(O)OR¹⁹, -C(O)NH₂, -C(O)NHR²⁰, ou -C(O)NR²⁰R²¹ ;
R⁶ et R¹⁰ sont indépendamment un atome d'hydrogène ou -R²³;
R⁷ est =O, =NOH, =NOR^{P}, ou =NOR²⁴ ;
un parmi R⁸ et R⁹ est un atome d'hydrogène, et l'autre est -OH ou -OR³² ; ou
R⁸ et R⁹ sont ensemble =O ;
R¹¹, R¹⁵, R¹⁹, R²⁴, et R²⁶ sont indépendamment un groupe alkyle, -(CH₂)alcényle, -(CH₂)alcynyle, alkyle substitué par un ou deux ou trois substituants indépendamment choisis parmi un groupe cycloalkyle, halo, aryle, hétéroaryle, et hétérocyclyle, -(CH₂)alcényle substitué par un ou deux ou trois substituants indépendamment choisis parmi un groupe cycloalkyle, halo, aryle, hétéroaryle et hétérocyclyle ou -(CH₂)alcynyle substitué par un ou deux ou trois substituants indépendamment choisis parmi un groupe cycloalkyle, aryle, hétéroaryle et hétérocyclyle ;
R¹², R¹³, R²⁰, R²¹, R²⁷, et R²⁸ sont indépendamment un groupe alkyle, cycloalkyle, -(CH₂)alcényle, -(CH₂)alcynyle, cycloalkyle, aryle, hétéroaryle, hétérocyclyle, alkyle substitué par un substituant choisi parmi un groupe cycloalkyle, aryle, hétéroaryle, hétérocyclyle, -NH₂, -NHR³⁰ et -NR³⁰R³¹, -(CH₂)alcényle substitué par un substituant choisi parmi un groupe cycloalkyle, aryle, hétéroaryle, hétérocyclyle, -NH₂, -NHR³⁰ et -NR³⁰R³¹ ou -(CH₂)alcynyle substitué par un substituant choisi parmi un groupe cycloalkyle, aryle, hétéroaryle, hétérocyclyle, -NH₂, -NHR³⁰ et -NR³⁰R³¹ ;
R²³ est un groupe alkyle, alcényle, alcynyle, aryle, hétéroaryle, hétérocyclyle, alkyle substitué par un ou deux ou trois substituants indépendamment choisis parmi un groupe cycloalkyle, halo, aryle, hétéroaryle et hétérocyclyle, alcényle substitué par un ou deux ou trois substituants indépendamment choisis parmi un groupe cycloalkyle, halo, aryle, hétéroaryle et hétérocyclyle, ou alcynyle substitué par un ou deux ou trois substituants indépendamment choisis parmi un groupe cycloalkyle, aryle, hétéroaryle et hétérocyclyle ;
R³⁰ et R³¹ sont indépendamment un groupe alkyle, cycloalkyle, aryle, hétéroaryle, hétérocyclyle, -(CH₂)alcényle, -(CH₂)alcynyle, cycloalkyle, alkyle substitué par un substituant choisi parmi un groupe cycloalkyle, aryle, hétéroaryle, hétérocyclyle, -NH₂, -NH(alkyle) et -N(alkyle)₂, -(CH₂)alcényle substitué par un substituant choisi parmi un groupe cycloalkyle, aryle, hétéroaryle, hétérocyclyle, -NH₂, -NH(alkyle) et -N(alkyle)₂ ou -(CH₂)alcynyle substitué par un substituant choisi parmi un groupe cycloalkyle, aryle, hétéroaryle, hétérocyclyle, -NH₂, -NH(alkyle) et -N(alkyle)₂
R³² est -R²⁶, -C(O)OR²⁶, -C(O)NH₂, -C(O)NHR²⁷, ou -C(O)NR²⁷R²⁸ ; et
X¹ est un atome d'hydrogène ou un fluorure.

2. Composé selon la revendication 1 présentant la formule (I) ou la formule (II), ou sel pharmaceutiquement acceptable de celui-ci, dans lequel R¹ est le groupe méthyle, prop-2-ynyle, 3-(5-pyridin-2-ylthién-2-yl)prop-2-ynyle, 3-(quinoléin-3-yl)prop-2-ényle, 3-(3-pyridin-2-ylisoxazol-5-yl)prop-2-ynyle, ou 3-(5-pyrimidin-2-ylthién-2-yl)prop-2-ynyle ; R² est un atome d'hydrogène ; R³ est -OH, le groupe ((phényl)carbonyl)oxy, ((méthyl)carbonyl)oxy, (triméthylsilyl)oxy, ou (triéthylsilyl)oxy et R⁴ est un atome d'hydrogène, ou R³ et R⁴ sont ensemble =O ou -CH₂O- ; R⁵ est un atome d'hydrogène, le groupe méthyle, éthyle, propyle, prop-2-ynyle, prop-2-ényle, phénylméthyle, 4-méthoxyphénylméthyle, ou 2,4-diméthoxyphénylméthyle ; R⁶ est un atome d'hydrogène, le groupe méthyle, éthyle, éthényle, ou phényle ; R⁷ est =O ; R⁸ est un atome d'hydrogène et R⁹ est -OH, ou R⁸ et R⁹ sont ensemble =O ; R¹⁰ est un atome d'hydrogène, le groupe méthyle, éthyle, prop-2-ynyle ou prop-2-ényle ; et X¹ est un atome d'hydrogène ou un groupe fluorure.

3. Composé selon la revendication 1 présentant la formule (I) ou la formule (II), ou sel pharmaceutiquement acceptable de celui-ci, dans lequel R¹ est un groupe alkyle, -(CH₂)alcynyle ou -(CH₂)alcynyle substitué par un groupe thiényle, dans lequel le groupe thiényle est substitué par un groupe pyridyle ; R² est un atome d'hydrogène ; R³ est le groupe ((phényl)carbonyl)oxy ; R⁴ est un atome d'hydrogène ; R⁵ est un atome d'hydrogène ou un groupe alkyle ; R⁶ est un atome d'hydrogène ; R⁷ est =O ; R⁸ et R⁹ sont ensemble =O ; R¹⁰ est un atome d'hydrogène ; et X¹ est un atome d'hydrogène.

4. Composé selon la revendication 1 ayant la formule (I) ou la formule (II), ou sel pharmaceutiquement acceptable de celui-ci, dans lequel R¹ est le groupe méthyle, prop-2-ynyle ou prop-2-ynyle substitué par un groupe thiényle, dans lequel le groupe thiényle est substitué par un groupe pyridyle ; R² est un atome d'hydrogène ; R³ est le groupe ((phényl)carbonyl)oxy ; R⁴ est un atome d'hydrogène ; R⁵ est un atome d'hydrogène ou le groupe méthyle ; R⁶ est un atome d'hydrogène ; R⁷ est =O ; R⁸ et R⁹ sont ensemble =O ; R¹⁰ est un atome d'hydrogène ; et X¹ est un atome d'hydrogène.

5. Composé selon la revendication 3 ayant la formule (I) ou la formule (II), ou sel pharmaceutiquement acceptable de celui-ci, dans lequel R¹ est le groupe méthyle ; R² est un atome d'hydrogène ; R³ est le groupe ((phényl)carbonyl)oxy ; R⁴ est un atome d'hydrogène ; R⁵ est un atome d'hydrogène ou le groupe méthyle ; R⁶ est un atome d'hydrogène ; R⁷ est =O ; R⁸ et R⁹ sont ensemble =O ; R¹⁰ est un atome d'hydrogène ; et X¹ est un atome d'hydrogène.

6. Composé selon la revendication 3 ayant la formule (I) ou la formule (II), ou sel pharmaceutiquement acceptable de celui-ci, dans lequel R¹ est le groupe prop-2-ynyle ; R² est un atome d'hydrogène ; R³ est le groupe ((phényl)carbonyl)oxy ; R⁴ est un atome d'hydrogène ; R⁵ est un atome d'hydrogène ou le groupe méthyle ; R⁶ est un atome d'hydrogène ; R⁷ est =O ; R⁸ et R⁹ sont ensemble =O ; R¹⁰ est un atome d'hydrogène ; et X¹ est un atome d'hydrogène.

7. Composé selon la revendication 3 ayant la formule (I) ou la formule (II), ou sel pharmaceutiquement acceptable de celui-ci, dans lequel R¹ est le groupe 3-(5-pyridin-2-ylthién-2-yl)prop-2-ynyle ; R² est un atome d'hydrogène ; R³ est le groupe ((phényl)carbonyl)oxy ; R⁴ est un atome d'hydrogène ; R⁵ est un atome d'hydrogène ou le groupe méthyle ; R⁶ est un atome d'hydrogène ; R⁷ est =O ; R⁸ et R⁹ sont ensemble =O ; R¹⁰ est un atome d'hydrogène ; et X¹ est un atome d'hydrogène.

8. Composition destinée à la prophylaxie ou au traitement d'infections bactériennes chez un poisson ou un mammifère comprenant une quantité thérapeutiquement efficace d'un composé selon la revendication 1 et un excipient.

9. Utilisation d'un composé selon la revendication 1 pour la préparation d'un médicament destiné à la prophylaxie ou au traitement d'infections bactériennes.

10. Composé selon la revendication 1 ou sel pharmaceutiquement acceptable de celui-ci, lequel est
le 3,4,6-tridéoxy-3-(diméthylamino)-β-D-xylo-hexopyranoside de (2aR,4aS,6R,8S,9R,10R,12R,15R,15aS,15bS)-15-éthyl-3,4a,6,8,10,12,15a-heptaméthyl-2,5,11,13-tétraoxo-8-((3-(5-(pyridin-2-yl)thién-2-yl)prop-2-ynyl)oxy)hexadécahydro-2H-1,14-dioxa-3-azacyclotétradéca(1,2,3-cd)pentalén-9-yle ;
le 3,4,6-tridéoxy-3-(diméthylamino)-β-D-xylo-hexopyranoside de (2aR,4aS,6R,8S,9R,10R,12R,15R,15aS,15bS)-15-éthyl-8-méthoxy-3,4a,6,8,10,12,15a-heptaméthyl-2,5,11,13-tétraoxohexadécahydro-2H-1,14-dioxa-3-azacyclotétradéca(1,2,3-cd)pentalén-9-yle;
le 3,4,6-tridéoxy-3-(diméthylamino)-β-D-xylo-hexopyranoside de (2aS,4aR,6R,8S,9R,10R,12R,15R,15aS,15bR)-15-éthyl-8-méthoxy-3,4a,6,8,10,12,15a-heptaméthyl-2,5,11,13-tétraoxohexadécahydro-2H-1,14-dioxa-3-azacyclotétradéca(1,2,3-cd)pentatén-9-yle ;
le 4-O-benzoyl-2,6-didéoxy-3-C-méthyl-3-O-méthyl-α-L-ribohexopyranoside de (2aR,4aS,6R,8S,9R,10S,11S,12R,15R,15aS,15bS)-15-éthyl-3,4a,6,8,10,12,15a-heptaméthyl-2,5,13-trioxo-8-(prop-2-ynyloxy)-9-((3,4,6-tridéoxy-3-(diméthylamino)-β-D-xylohexopyranosyl)oxy)hexadécahydro-2H-1,14-dioxa-3-azacyclotétradéca(1,2,3-cd)pentalén-11-yle ;
le 3,4,6-tridéoxy-3-(diméthylamino)-β-D-xylo-hexopyranoside de (2aR,4aS,6R,8S,9R,10R,12R,15R,15aS,15bS)-15-éthyl-3,4a,6,8,10,12,15a-heptaméthyl-2,5,11,13-tétraoxo-8-(prop-2-ynyloxy)-hexadécahydro-2H-1,14-dioxa-3-azacyclotétradéca(1,2,3-cd)pentalén-9-yle ;
le 3,4,6-tridéoxy-3-(diméthylamino)-β-D-xylo-hexopyranoside de (2aR,4aS,6R,8S,9R,10R,12R,15R,15aS,15bS)-15-éthyl-8-méthoxy-4a,6,8,10,12,15a-hexaméthyl-2,5,11,13-tétraoxohexadécahydro-2H-1,14-dioxa-3-azacyclotétradéca(1,2,3-cd)pentalén-9-yle.
